# FASCICULE DE BREVET EUROPEEN

(11) **EP 1 648 883 B1**
(45) Date de publication et mention de la délivrance du brevet: **30.08.2017**
(21) Numéro de dépôt: 04785982.2
(22) Date de dépôt: 20.07.2004
(51) Int. Cl.: C07D 401/14, C07F 17/00, C09K 11/06, G01N 33/58, C07D 519/00, C07F 9/58

(54) **COMPLEXES DE LANTHANIDES, LEUR PREPARATION ET LEURS UTILISATIONS**
HERSTELLUNG VON LANTHANIDKOMPLEXEN UND DEREN VERWENDUNGEN
LANTHANIDE COMPLEXES PREPARATION AND USES THEREOF

(30) Priorité: 25.07.2003 FR 0309158
(43) Date de publication de la demande: 26.04.2006
(73) Titulaire: CENTRE NATIONAL DE LA RECHERCHE SCIENTIFIQUE, 75016 Paris (FR); UNIVERSITE LOUIS PASTEUR DE STRASBOURG, 67000 Strasbourg (FR)
(72) Inventeur: CHARBONNIERE, Loic, F-67720 Weyersheim (FR); ZIESSEL, Raymond, F-67400 Souffelweysheim (FR); WEIBEL, Nicolas, F-68000 Colmar (FR); RODA, Aldo, I-40123 Bologna (IT); GUARDIGLI, Massimo, I-48010 Roncalceci (IT)
(74) Mandataire: Goulard, Sophie
(86) Numéro de dépôt international: PCT/FR2004/001921
(87) Numéro de publication internationale: WO 2005/014581

(56) Documents cités:
- EP-A- 0 493 745
- EP-A- 0 649 020
- EP-A- 0 770 610
- WO-A1-02/059097
- US-A- 5 216 134
- US-A- 5 559 214
- US-A- 5 892 029
- US-A1- 2002 090 342
- US-B1- 6 509 324
- N. WEIBEL ET AL.: "Engineering of Highly Luminescent Lanthanide Tags Suitable for Protein Labeling and Time-Resolved Luminescence Imaging" J. AM. CHEM. SOC., vol. 126, 2004, pages 4888-4896, XP002315969

## Description

La présente invention concerne des composés capables de former des complexes avec les lanthanides, les complexes obtenus et leurs utilisations.

Les marqueurs radioactifs ont été largement utilisés dans le domaine de l'imagerie médicale et de l'immunologie. En raison des inconvénients qu'ils présentent, ils ont été remplacés en grande partie par des marqueurs fluorescents.

Cependant l'utilisation des marqueurs fluorescents présente quelques inconvénients, notamment dus à l'auto-fluorescence des milieux biologiques étudiés et à la diffusion de lumière dans les appareillages. Les complexes d'ions lanthanides ont été proposés pour permettre une acquisition en temps résolu qui supprime ces inconvénients. Pour être utilisé comme marqueur luminescent en temps résolu, un complexe d'ion lanthanide doit présenter de nombreuses caractéristiques, parmi lesquelles les plus importantes sont l'hydrophilicité, la stabilité dans l'eau, la présence de chromophores capables de générer l'effet d'antenne (Sabbatini, N. et al. Coord. Chem. Rev. 1990, 123, 201), de bonnes propriétés photo-physiques (absorption élevée, excitation dans une gamme énergétique facilement accessible, temps de vie de l'état excité élevé et rendement quantique de luminescence élevé) et une fonction réactive qui permet un greffage covalent.

Les composés actuellement proposés possèdent rarement la totalité de ces critères. Par exemple, les premiers complexes développés par la firme Wallac Oy sous l'appellation Delfia Chelate (Hemmilä, I. et al. Anal. Biochem. 1984, 137, 335) ne possèdent pas de bonnes propriétés photo-physiques et il est nécessaire de procéder à une étape d'extraction du lanthanide pour mesurer sa luminescence. Les composés développés par CIS Bio international sont des cryptates qui nécessitent l'utilisation d'anions fluorures pour augmenter la luminescence (Hemmilâ, I. et al. Drug Discovery Today, 1997, 2, 373). La stabilité des composés pose également de graves problèmes. Ainsi les composés développés par CyberFluor sous le nom de BCPDA ne forment des complexes stables luminescents qu'à fortes concentrations (Marriott, G. et al., Biophysical Journal, 1994, 67, 957).

Des complexes de lanthanide, notamment de gadolinium, ont été utilisés comme agents de relaxation ou de contraste pour l'imagerie médicale par RMN (Caravan, P. et al. Chem. Rev. 1999, 99, 2293). Cette utilisation est permise par le fait que la première sphère de coordination du lanthanide n'est pas complètement saturée par le ligand en solution aqueuse, des molécules d'eau pouvant alors compléter la sphère de coordination.

Le but de la présente invention est de proposer des complexes de lanthanide qui présentent des propriétés améliorées par rapport aux complexes de lanthanide de l'art antérieur. C'est pourquoi l'invention a pour objet de nouveaux composés, leur utilisation pour la préparation de complexes avec des ions lanthanides, ainsi que l'utilisation des complexes obtenus comme marqueurs fluorescents, comme agents de relaxation pour la RMN, ou pour l'imagerie RMN.

Un composé selon la présente invention répond à la formule (I) : 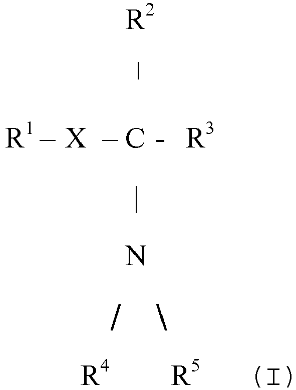 sous forme cationique, zwitterionique ou anionique, dans laquelle
- R¹ est un groupe choisi parmi les groupes carboxyle, ester N-succinimidique et ester sulfosuccinimidique ;
- X représente une simple liaison ou un groupement alkylène ayant 2 atomes de carbone ;
- R² est un groupement anionique à pH neutre A² choisi parmi les groupes CO₂H, SO₃H, P(O)(OR)OH, P(O)R(OH) et P(O)(OH)₂ dans lesquels R est un groupe alkyle ou un groupe aryle ;
- R³ représente H ;
- le substituant R⁴ comprend un groupement 2,2'-bipyridyle portant un groupe A⁴ choisi parmi un carboxyle, un monoalkylphosphonate, un monoarylphosphonate et un phosphonyle, ou un groupement phénanthrolinyle portant un groupe A⁴ carboxyle, le substituant R⁴ correspondant à l'une des formules (II), (III), (IV) et (V) suivantes : 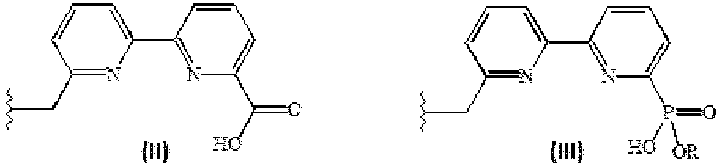 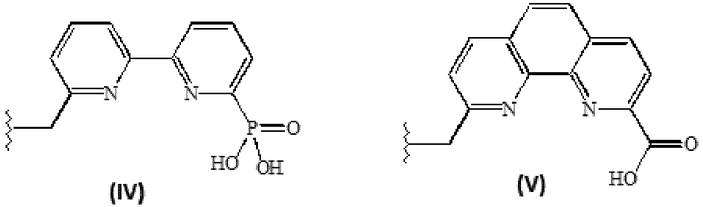 R étant aryle ou alkyle ;
- le substituant R⁵ est identique à R⁴, chacun des substituants R⁴ et R⁵ étant un substituant monovalent, les substituants R⁴ et R⁵ ne formant pas ensemble un groupe divalent.

Dans les substituants R², R⁴ et R⁵, R est un groupe alkyle en C₁ à C₃ ou un groupe aryle en C₅ à C₉.

Le composé selon l'invention peut également répondre à la formule (I) : 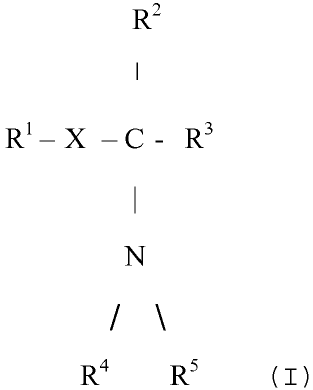 dans laquelle R¹ et R² sont -COOH, X est -CH₂-CH₂, R³ représente H, R⁴ et R⁵ comprennent chacun un groupement méthoxyphénanthrolinyle portant un groupe A⁴ carboxyle, les substituants R⁴ et R⁵ correspondant à la formule (VI) ci-après :

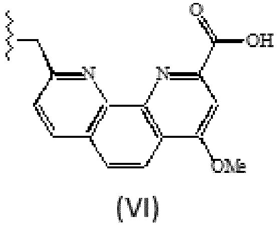

Le substituant R⁴ est un substituant qui a des propriétés d'absorption de lumière et qui permet de former trois cycles chélates avec un lanthanide.

Le substituant R⁵ est un substituant qui permet de former trois cycles chélates avec un lanthanide.

Telle qu'utilisée ici, l'expression "groupe qui est anionique à pH neutre" signifie un groupe fonctionnel qui, à pH neutre, se trouve sous forme anionique, c'est-à-dire porteur d'une charge négative. Dans un composé de l'invention, les groupes anioniques à pH neutre A², peuvent être choisis indépendamment les uns des autres parmi les groupes -CO₂H, -SO₃H, -P(O)(OR)OH, -P(O)R(OH) et -P(O)(OH)₂ dans lesquels R est un groupe alkyle en C₁ à C₃ ou un groupe aryle en C₅ à C₉. Suivant le pH du milieu réactionnel, les composés (I) sont obtenus sous forme cationique, zwitterionique ou anionique. En milieu acide, l'azote porteur des substituants R⁴ et R⁵, ainsi qu'éventuellement les hétéroatomes de ces substituants, se trouvent sous forme protonée et le composé est sous forme cationique. En milieu basique, les différents groupes Aⁱ se présentent sous forme de sels et le composé est sous forme anionique. A des pH intermédiaires, de l'ordre de 6 à 8, le composé se présente sous forme zwitterionique.

Un complexe selon la présente invention est constitué par un ion lanthanide Ln complexé par un ligand qui répond à la formule (I) ci-dessus. L'ion lanthanide est choisi parmi les ions europium, terbium, samarium, dysprosium, erbium, ytterbium, néodyme et gadolinium. On utilisera de préférence l'europium, le terbium, le samarium ou le dysprosium si le complexe est destiné à être utilisé pour le marquage par fluorescence, et l'europium, le dysprosium ou le gadolinium lorsque le complexe est destiné à être utilisé comme agent de contraste pour l'IRMN.

Dans un complexe selon l'invention le substituant R⁴ forme 3 cycles chélates avec le cation lanthanide ainsi que le substituant R⁵ qui forme 3 cycles chélates à 5 membres avec le cation lanthanide.

Un composé (I) peut être obtenu par des procédés bien connus de l'homme de métier à partir de produits commerciaux ou décrits dans la littérature par le schéma suivant : 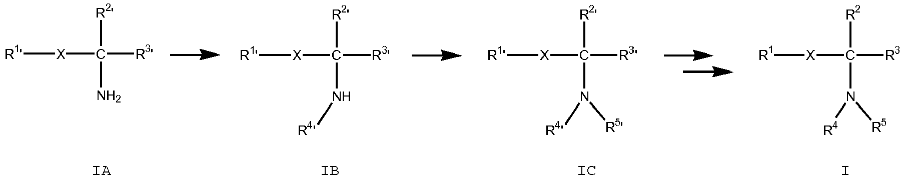 dans lequel X, R¹, R², R³, R⁴ et R⁵ ont la signification donnée précédemment, et R^{1'}, R^{2'}, R^{3'}, R⁴' et R^{5'} représentent des groupements précurseurs de R¹, R², R³, R⁴ et R⁵ respectivement.

Au cours des deux premières étapes, on introduit successivement les groupements R⁴' et R⁵' sur une molécule IA contenant X et les groupes R¹', R²' et R³' pour obtenir le composé IC.

Au cours d'étapes ultérieures, on transforme les groupes R¹', R²', R³', R⁴' et R⁵' du composé IC respectivement en groupes R¹, R², R³, R⁴ et R⁵.

Les groupements R⁴' et R⁵' étant identiques en vue d'obtenir des groupements R⁴ et R⁵ identiques, ils sont introduits simultanément au cours de la première étape.

Lorsque le composé (I) est un composé dans lequel les groupements R¹ et R² sont des fonctions carboxyles, le groupement R³ est un atome d'hydrogène et le groupement X est une simple liaison, ou un groupe éthylène, on choisira avantageusement comme produit de départ IA respectivement le diester éthylique de l'acide aminomalonique, et le diester méthylique de l'acide glutamique, qui sont des produits disponibles dans le commerce.

Lorsque les groupements R⁴ et R⁵ sont dérivés de la 2,2'-bipyridine, on fait réagir le produit de départ au cours de la première étape, avec la 6-bromométhyl-6'-bromo-2,2'-bipyridine pour obtenir un composé dibromé IC. La 6-bromométhyl-6'-bromo-2,2'-bipyridine peut être obtenue par une réaction de bromation radicalaire de la 6-méthyl-6'-bromo-2,2'-bipyridine par la N-bromo-succinimide dans le benzène, la 6-méthyl-6'-bromo-2,2'-bipyridine étant obtenue selon la méthode décrite par Houghton M. et al, J. Chem. Soc., Dalton Trans. 1997, 2725. Le schéma réactionnel de la première étape de ce cas particulier est donné ci-après.

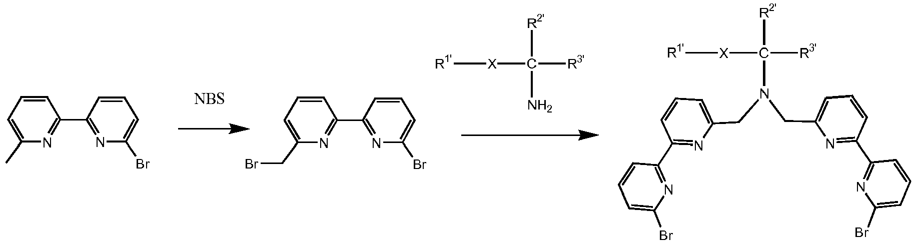

Lorsque le composé dibromé IC est soumis à une carboalkoxylation, suivie d'une saponification avec NaOH et d'une acidification avec HCl, on obtient un composé (I) dans lequel les groupements A⁴ sont des groupes carboxyles. La carboalkoxylation peut être effectuée suivant le procédé décrit par El-Ghayoury et al, J. Org. Chem., 2000, 65, 7757.

Lorsqu'on fait réagir le composé dibromé IC avec le dialkylphosphite (selon la méthode décrite par Penicaud et al, Tetrahedron Lett. 1998, 39, 3689), on obtient le dialkylester de l'acide phosphonique, chaque atome de brome étant remplacé par un groupe P(O)(OR)₂. Le dialkylester de l'acide phosphonique donne, par une saponification avec NaOH dans l'eau, suivie d'une acidification avec HCl, un composé (I) dans lequel les groupements A⁴ sont des groupements P(O)(OH)OR.

Par réaction du dialkylester de l'acide phosphonique P(O)(OR)₂ avec le bromure de triméthylsilyle suivie d'une hydrolyse (selon la méthode décrite par McKenna C. et al., Tetrahedron Lett, 1977, 18, 155), on obtient un composé (I) dans lequel les deux groupes anioniques A⁴ sont des groupements P(O)(OH)₂. Le même résultat peut être obtenu par une hydrolyse acide par HCl du dialkylester de l'acide phosphonique P(O)(OR)₂.

Le schéma réactionnel des trois modes de mise en oeuvre ci-dessus est donné ci-après.

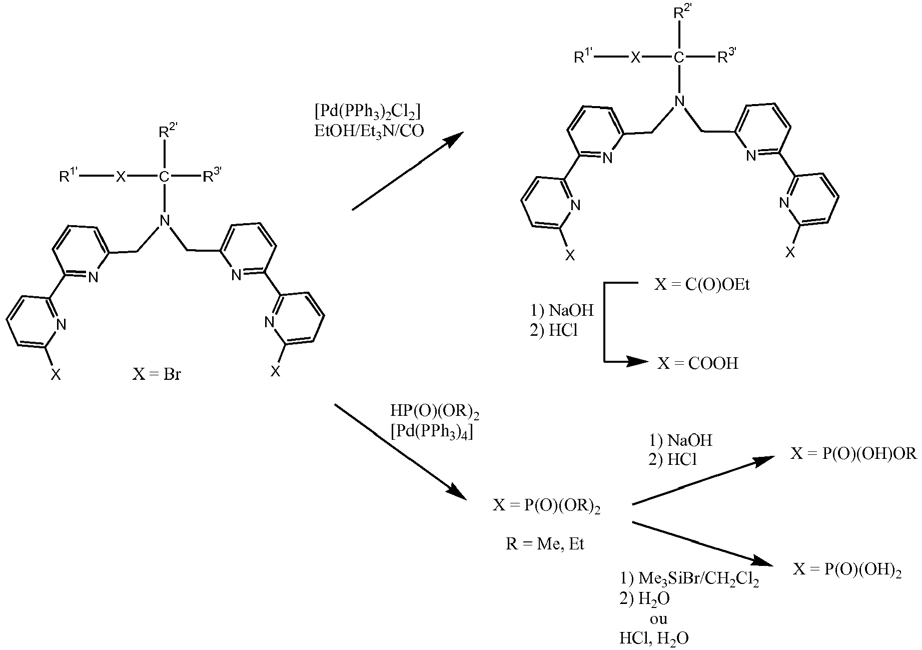

Lorsque les groupements R⁴ et R⁵ sont dérivés de la 1,10-phénantroline, on fait réagir le produit de départ au cours de la première étape avec la 2-bromométhyl-9-éthoxycarbonyl-1,10-phénantroline. La préparation de la 2-bromométhyl-9-éthoxycarbonyl-1,10-phénantroline est décrite par Ulrich G. et al, (Tetrahedron Lett. 2001, 42, 6113). En soumettant le composé diester obtenu, à une saponification avec NaOH, suivie d'une acidification avec HCl dilué, on obtient un composé (I) dans lequel les groupements A⁴ sont des carboxyles. Le schéma réactionnel est donné ci-après.

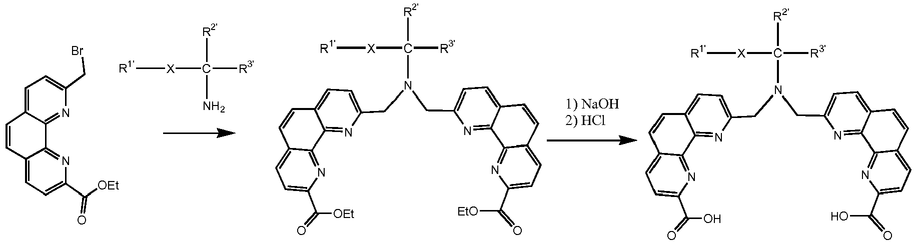

Un substituant R¹ souhaité peut être obtenu en choisissant soit un composé de départ qui le porte, soit un composé de départ qui porte un précurseur R¹' du substituant souhaité. Lorsqu'un substituant R¹ est obtenu à partir d'un précurseur R¹', la formation du substituant souhaité peut se faire sur un composé de formule (IC) contenant le précurseur ou sur un complexe formé avec un cation lanthanide et un composé de formule (I) contenant le précurseur.

Un substituant R¹ du type carboxyle peut être obtenu par une réaction de saponification à partir d'un groupe précurseur R¹' contenant une fonction ester carboxylique.

Un substituant R¹ du type ester N-succinimidique peut être obtenu à partir d'un complexe par activation d'un précurseur carboxyle avec de la N-(3-diméthylaminopropyl)-N'-éthylcarbodiimide suivie d'une réaction avec la N-hydroxy-succinimide.

Un complexe selon l'invention peut être obtenu par réaction d'un composé donneur de cation lanthanide avec un composé de formule (I). Comme exemple de composés donneurs de cation lanthanide, on peut citer les halogénures de lanthanide hydratés, les nitrates de lanthanide hydratés, les carbonates de lanthanide et les triflates de lanthanide. La réaction est effectuée en solution dans un solvant. Le solvant est choisi de préférence parmi l'eau, le méthanol, l'éthanol ou l'acétonitrile.

Dans un mode de réalisation préféré, on fait réagir le composé (I) avec le précurseur de l'ion lanthanide dans un mélange de méthanol et d'eau à un pH allant de 3 à 5, pendant une durée comprise entre 10 minutes et 24 heures, à une température comprise entre 25°C à 80°C. Ensuite, le pH de la solution est porté à 7,0 et le méthanol est évaporé avant d'isoler le complexe formé.

Les complexes de la présente invention peuvent être utilisés notamment pour le marquage par fluorescence ou pour l'imagerie par résonnance magnétique nucléaire. Pour ces applications, les groupes R¹ préférés sont les groupes carboxyle (qui doivent être activés avant le couplage covalent avec la molécule à marquer), et les groupes ester N-succinimidique (qui peuvent se lier directement avec la molécule à marquer).

Les complexes de la présente invention sont utiles pour des analyses ou des dosages de composés par marquage des composés. Le procédé consiste à lier de manière covalente au composé à doser, un marqueur constitué par un complexe selon l'invention, et à détecter ou quantifier la présence du composé marqué grâce aux propriétés de luminescence du marqueur. Les complexes d'europium, de terbium, de samarium ou de dysprosium sont particulièrement préférés pour cette application.

Lorsque les complexes d'ions lanthanides selon l'invention sont destinés à être utilisés comme agents de relaxation pour la résonnance magnétique nucléaire, on utilise de préférence des complexes de gadolinium, d'europium ou de dysprosium.

La présente invention sera décrite plus en détail par les exemples donnés ci-après à titre d'illustration, auxquels elle n'est cependant pas limitée.

### Exemple 1

### Préparation du composé 1

Le composé **1** a été obtenu selon le schéma synthétique suivant. L'isomère (S) de l'ester glutamique choisi pourrait être remplacé par l'isomère (R) ou un mélange des deux isomères.

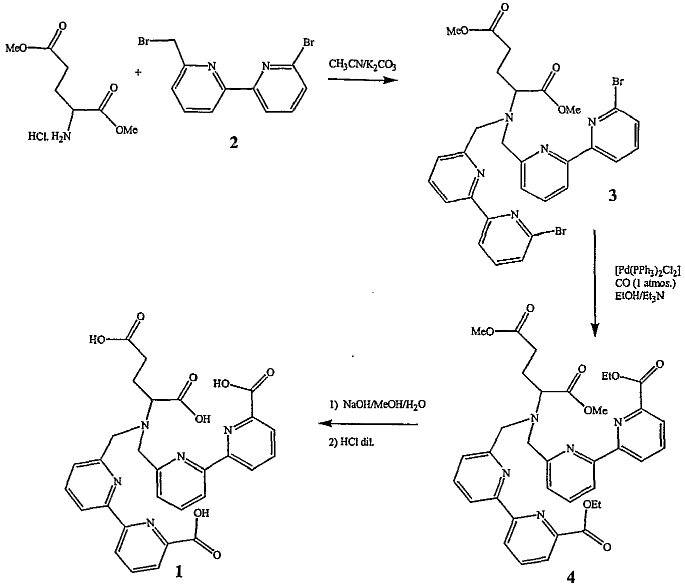

### Préparation du composé 2

Le composé **2** a été préparé selon le procédé décrit par S. Mameri, et al., dans Synthesis, 2003, 17, 2713. Dans un ballon de 250 mL, on introduit 1,5 g (6,0 mmol) de 6-méthyl-6'-bromo-2,2'-bipyridine, 66 mg (0,4 mmol) d'azo-bis-isobutyronitrile (AIBN), et 1,3 g (7,3 mmol) de N-bromosuccinimide dans 90 mL de benzène. La solution est chauffée à reflux pendant 2h30 en l'irradiant avec une lampe halogène standard de 100 W. Le solvant est évaporé sous pression réduite et le résidu solide est chromatographié sur silice en utilisant un gradiant de CH₂Cl₂/hexane de 50/50 à 100/0. On obtient 940 mg (2,9 mmol) de composé **2** (correspondant à un rendement de 48%) qui présente les caractéristiques suivantes :

*R_{f}* = 0,42, SiO₂, CH₂Cl₂.

¹H-RMN (CDCl₃, 200 MHz) : δ 4,61 (s, 2H), 7,48 (d, 1H, ³J=7,5 Hz), 7,50 (d, 1H, ³J=7,5 Hz), 7,68 (t, 2H, ³J=8,0 Hz), 7,83 (t, 1H, ³J=8,0 Hz), 8,33 (d, 1H, ³J=8,0 Hz), 8,44 (d, 1H, ³J=8,0 Hz).
¹³C-RMN (CDCl₃, 50 MHz) : δ 34,0, 120,1, 120,7, 124,0, 128,2, 138,1, 139,3, 141,6, 154,3, 156,4, 156,9.
Analyses calculées pour C₁₁H₈N₂Br₂ . C 40,28, H 2,46, N 8,54. Trouvées : C 40,12, H 2,34, N 8,44.
FAB⁺/MS : 327 (50%), 329 (100%), 3.31 (50%, [2+H]⁺) .

### Préparation du composé 3

Dans un tube de Schlenk sous atmosphère d'argon, on introduit 470 mg (2,22 mmol) de chlorhydrate du diméthyle ester de l'acide L-glutamique et 1,23 g de K₂CO₃ (8,90 mmol) dans 100 mL d'acétonitrile fraîchement distillée sur P₂O₅. La solution est chauffée à 80°C pendant 30 minutes. On ajoute 1,60 g (4,88 mmol) de composé **2** et on chauffe pendant 23 heures à 80°C. La solution est évaporée à sec, le résidu est redissous avec 100 mL de CH₂Cl₂ et 20 mL d'eau. La phase aqueuse est lavée avec deux portions de 20 mL de CH₂Cl₂ et la somme des phases organiques est séchée sur MgSO₄, filtrée, puis évaporée à sec. Le résidu solide est soumis à une chromatographie flash sur silice (φ=5 cm, h=12 cm) avec un mélange de CH₂Cl₂/MeOH (100/0 à 97/3) comme éluant. On obtient 995 mg (1,49 mmol) de composé **3** (correspondant à un rendement de 67%) qui présente les caractéristiques suivantes :

*R_{f}* = 0,34, SiO₂, CH₂Cl₂/MeOH (98/2).

¹H-RMN (CDCl₃, 200 MHz) : δ 2,06-2,20 (m, 2H), 2,39-2,68 (m, 2H), 3,50 (s, 3H), 3,54-3,62 (m, 1H), 3,76 (s, 3H), 3,99-4,16 (m, 4H), 7,43-7,48 (m, 4H), 7,63 (t, 2H, ³J=8,0 Hz), 7,71 (t, 2H, ³J=8,0 Hz), 8,23 (d, 2H, ³J=8,0 Hz), 8,39 (d, 2H, ³J=8,0 Hz).
¹³C-RMN (CDCl₃, 50 MHz) : δ 24,8, 30,3, 51,5, 57,2, 62,1, 119,6, 119,7, 123,5, 127,8, 137,3, 139,1, 141,5, 153,8, 157,4, 159,1, 173,1, 173,4.
Analyses calculées pour C₂₉H₂₇N₅O₄Br₂ : C 52,04, H 4,07, N 10,46. Trouvées : C 51,81, H 3,85, N 10,19.
FAB⁺/MS : 670,2 ([3+H]⁺, 100%).

### Préparation du composé 4

Dans un ballon bicol de 250 mL, on introduit 995 mg (1,49 mmol) de composé **3** et 150 mg (0,21 mmol) de [Pd(PPh₃)₂Cl₂] dans 50 mL d'éthanol et 50 mL de triéthyl-amine. La solution est chauffée à 70°C pendant 15 heures en faisant barboter un flux de CO. La solution est évaporée à sec, le solide obtenu est redissous dans 100 mL de CH₂Cl₂, filtré sur célite, puis la phase organique est extraite avec 20 mL d'eau. La phase aqueuse est lavée avec deux portions de 20 mL de CH₂Cl₂ et les phases organiques combinées sont séchées sur MgSO₄, filtrées puis évaporées à sec. Le résidu est soumis à une chromatographie flash sur silice (φ=5 cm, h=10 cm) avec un mélange de CH₂Cl_{2/}MeOH (99/1 à 90/10) comme éluant. On obtient 588 mg (0,90 mmol) de **4** sous forme d'une huile légèrement orangée (correspondant à un rendement de 60%), qui présente les caractéristiques suivantes :

*R_{f}* = 0,30, SiO₂, CH₂Cl₂/MeOH (95/5).

¹H-RMN (CDCl₃, 200 MHz) : δ 1,46 (t, 6H, ³J=7,0Hz), 2,06-2,19 (m, 2H), 2,38-2,65 (m, 2H), 3,49 (s, 3H), 3,55-3,63 (m, 1H), 3,76 (s, 3H), 4,02-4,19 (m, 4H), 4,48. (q, 4H, ³J=7,0 Hz), 7,47 (d, 2H, ³J=8,0 Hz), 7,75 (t, 2H, ³J=8,0 Hz), 7,92 (t, 2H, ³J=8,0 Hz), 8,10 (d, 2H, ³J=8,0 Hz), 8,40 (d, 2H, ³J=8,0 Hz), 8,62 (d, 2H, ³J=8,0 Hz).
¹³C-RMN (CDCl₃, 50 MHz) : δ 14,3, 24,8, 30,4, 51,5, 57,2, 61,8, 62,0, 119,9, 123,5, 124,2, 124,8, 137,3, 137,7, 147,8, 154,6, 156,5, 159,0, 165,4, 173,2, 173,5.
Analyses calculées pour C₃₅H₃₇N₅O₈ : C 64,11, H 5,69, N 10,68. Trouvées : C 64,07, H 5,55, N 10,53.
FAB⁺/MS : 656,2 ([**4**+H]⁺, 100%).

### Préparation du composé 1

Dans un ballon équipé d'un réfrigérant, 588 mg (0,90 mmol) de **4** et 144 mg (3,60 mmol) de NaOH sont dissous dans un mélange de 50 mL de MeOH et 15 mL d'eau. Le mélange est chauffé à 70°C pendant 5 heures. La solution est évaporée à sec et le solide est dissous dans 10 mL d'eau sur lesquels on ajoute lentement une solution d'HCl 2N jusqu'à pH = 2-3. Le précipité qui se forme est isolé par centrifugation et séché sous vide. On obtient 411 mg (0,60 mmol) de composé **1** sous forme d'hydrochlorure **1**.3HCl jaune pâle (correspondant à un rendement de 67%) dont les caractéristiques sont les suivantes :
¹H-RMN (CD₃OD, 300 MHz) : δ 2,26-2,48 (m, 2H), 2,80-2,84 (m, 2H), 3,95-3,99 (m, 1H), 4,53-4,81 (m, 4H), 7,47 (d, 2H, ³J=7,5 Hz), 7,63 (t, 2H, ³J=8,0 Hz), 7,90 (t, 2H, ³J=8,0 Hz), 8,02 (d, 2H, ³J=7,5 Hz), 8,42 (d, 2H, ³J=7,5 Hz), 8,58 (d, 2H, ³J=7,5 Hz).
¹³C-RMN (CD₃OD, 75 MHz) : δ 23,1, 32,1, 57,0, 67,0, 122,3, 125,1, 125,9, 126,1, 139,7, 140,1, 149,0, 154,1, 155,5, 156,1, 168,0, 173,7, 176,4.
Analyses calculées pour C₂₉H₂₅N₅O₈.3HCl: C 51,15, H 4,14, N 10,28. Trouvées : C 51,01, H 4,43, N 9,95.
FAB⁺/MS : 572,5 ([1+H]⁺, 100%).

### Exemple 2

### Préparation du complexe 5 de formule [Eu.(1-4H).H₂O]Na

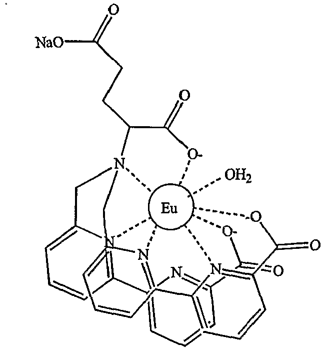

60 mg de **1**.3HCl (88 µmol) sont dissous dans un mélange de 30 mL de MeOH et de 30 mL d'eau. A cette solution est ajouté un mélange de 36 mg (98 µmol) de EuCl₃.₆H₂O dissous dans 3 mL de MeOH et 3 mL d'eau. La solution est chauffée à 70°C pendant 1 heure. Après refroidissement, le pH de la solution est remonté à 7,4 avec une solution à 5% de NaOH dans l'eau. La solution est concentrée à l'évaporateur rotatif jusqu'à apparition d'un léger trouble. Puis on ajoute du THF jusqu'à la formation d'un important précipité. Le précipité est isolé par centrifugation, puis séché sous vide pour donner 62 mg (74 µmol) de composé 5 (correspondant à un rendement de 85%) sous forme de solide beige dont les caractéristiques sont les suivantes :
¹H-RMN (D₂O/*t*-BuOH, 200 MHz, tous les signaux se présentent sous forme de singulets larges) : δ -9,40 (1H), -8,95 (1H),-4,23 (2H), -3,17 (1H), -2,21 (1H), 1,88 (1H), 2,73 (1H), 4,17 (1H), 6,06 (1H), 7,12 (1H), 7,80 (1-H), 7,88 (1H), 8,90 (1H), 9,60 (1H), 9,89 (1H), 11,08 (1H), 11,38 (1H), 12,01 (1H).
Analyses calculées pour C₂₉H₂₁NaN₅O₈Eu.5H₂O : C 41,84, H 3,75, N 8,41. Trouvées : C 41,93, H 3,62, N 8,44.
FAB⁺/MS : 720,2 (80%), 722,2 (100%), [**5**-H₂O-Na+2H]⁺.
IR (KBr, cm⁻¹) : 3420, 1619, 1574, 1460, 1384, 1274.

### Propriétés photo-physiques dans l'eau:

Absorption, λₘₐₓ [nm] (εₘₐₓ [M⁻¹.cm⁻¹]) : 320 (épaulement), 308 (19700), 276 (8700), 267 (9700), 253 (14400).
Emission : caractéristique des composés de l'europium avec des bandes fines à 581, 594, 615, 650 et 701 nm. Temps de vie de l'état excité : 0,62 ms. Rendement quantique (référence [Ru (bipy)₃]²⁺ dans l'eau) : 8%. Temps de vie de l'état excité dans l'eau lourde : 2,48 ms. Rendement quantique dans l'eau lourde : 35%.

### Exemple 3

### Préparation du complexe 6 de formule :

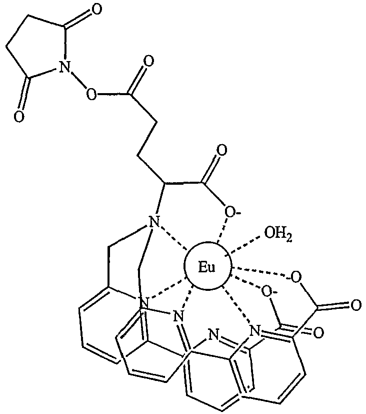

40 mg (48 µmol) de complexe **5** et 12 mg (63 µmol) de sels d'hydrochlorure d'éthyle-N,N-diméthyle-3-aminopropyle-carbodiimide (EDCI.HCl) sont mis en suspension dans 6 mL de DMSO. A cette solution, on ajoute 7,0 mg (61 µmol) de N-hydroxysuccinimide. La solution est agitée à température ambiante pendant 66 heures, durant lesquelles le complexe **5** se dissout, puis un précipité blanc se forme. Le solide est isolé par centrifugation et séché sous vide à 50°C pendant 2 heures. On obtient 31 mg (34 µmol) de **6** (correspondant à un rendement de 71%) dont les caractéristiques sont les suivantes :
Analyses calculées pour C₃₃H₂₅EuN₆O₁₀.5H₂O : C 43,67, H 3,89, N 9,26. Trouvées : C 43,60, H 3,80, N 9,16.
FAB⁺/MS : 720,1, 722,1 ([**6**-H₂O-C₄H₄NO₂+2H]⁺, 100%), 817,1, 819,1 ([**6**-H₂O+H]⁺, 30%).
IR (pastille de KBr, cm⁻¹) : 3420, 1739, 1629, 1573, 1459, 1384.

### Propriétés photo-physiques dans l'eau:

Absorption, λₘₐₓ [nm] (εₘₐₓ [M⁻¹.cm⁻¹]) : 320 (épaulement), 309 (20000), 276 (10000), 267 (10500), 253 (16000).
Emission : caractéristique des composés de l'europium avec des bandes fines à 581, 593, 615, 649 et 701 nm. Temps de vie de l'état excité : 0,63 ms. Rendement quantique (référence [Ru(bipy)₃]²⁺ dans l'eau) : 8%. Temps de vie de l'état excité dans l'eau lourde : 2,47 ms. Rendement quantique dans l'eau lourde : 34%.

### Exemple 4

### Marquage d'une amine par le complexe 5

10 mg de complexe **5** (13,1 µmol) sont mis en suspension dans 5 mL d'eau. On ajoute 3,5 mg (18,3 µmol) d'EDCI.HCl, puis 1,7 µL (13,2 µmol) de (+) -α-méthylbenzylamine. Après 15 minutes, puis une heure, on ajoute chaque fois 1,7 µL de (+)-α-méthylebenzylamine à température ambiante. L'agitation est poursuivie pendant 15 heures. La phase aqueuse est lavée avec 2 fois 10 mL de CH₂Cl₂, puis évaporée à sec, et l'on obtient 14 mg de solide jaune pâle. Après recristallisation avec un mélange MeOH/Et₂O, centrifugation et séchage sous vide, le complexe **7** (8,0 mg, 9,5 µmol) est récupéré sous forme de poudre crème (73%).
ISI-TOF/MS : 847,0513 ([7-H₂O+Na]⁺, 60 %), 825,0912 ([7-H₂O+H]⁺, 28%). La formule du complexe 7 est représentée ci-dessous.

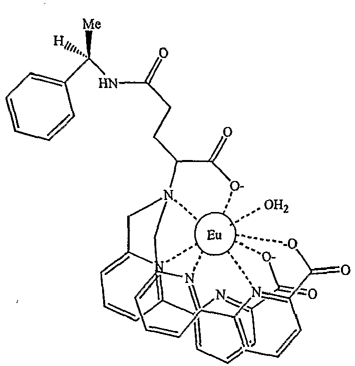

### Exemple 5

### Marquage de l'albumine sérique de boeuf ASB par le complexe 6

Le complexe **6** (2,0 mg) est ajouté à une solution d'ASB (5,4 mg) dans 1 mL de tampon borate (50 mM dans l'eau, pH = 7,0) afin d'obtenir un rapport molaire **6**/ASB de 30:1. La solution est agitée à température ambiante, conduisant à une dissolution complète de **6** après 2 heures. Après 24 heures d'agitation, la solution est déposée sur un filtre centrifuge (Centricon, Millipore, filtre à 30 KDa) et le volume de la solution est réduit à 200-300 µL par filtration. La solution est diluée avec 3 mL d'eau, puis le volume est réduit de nouveau à 200-300 µL par filtration. Cette dernière opération est répétée 3 à 4 fois, jusqu'à ce que les eaux de filtration ne soient plus luminescentes sous irradiation UV (absence d'europium). Les 200-300 µL de solution résiduelle contenant la protéine marquée et restant sur le filtre sont récupérés et stockés au frigidaire à 4°C.

### Caractérisation de l'ASB marquée

Le spectre d'absorption UV-Vis de la solution aqueuse d'ASB marquée montre une absorption intense due aux complexes d'europium, qui recouvre en partie l'absorption due à la protéine (λₘₐₓ = 278 nm, εₘₐₓ = 38000 M⁻¹.cm⁻¹). Par excitation de la solution dans la bande d'absorption des bipyridines (308 nm), on observe un spectre d'émission typique des composés d'europium, avec un temps de vie de l'état excité moyen de 1,1 ms (la décroissance n'est pas purement mono-exponentielle) et un rendement quantique de luminescence de 13%.

La caractérisation par spectrométrie de masse en mode MALDI-TOF (Matrice Assisted Laser Desorption Ionisation-Time Of Fly = Ionisation par désorption sur matrice assistée par Laser, analyse par temps de vol) s'effectue de la manière suivante. Une solution aqueuse d'ASB marquée est traitée à l'acide trifluoroacétique à 1% pour décomplexer l'europium, puis la protéine est adsorbée sur une colonne de chromatographie dont la phase solide hydrophobe est constituée de chaîne en C₄. Après lavage à l'eau, la protéine est relarguée avec de l'acétonitrile, puis analysée par MALDI-TOF (matrice d'acide α-cyano-4-hydroxycynnamique). La masse moyenne obtenue pour la protéine marquée sans europium est de 71700 Da (ASB, M = 66610 Da), conduisant à un rapport molaire marqueurs/BSA de 9/1 dans la protéine marquée.

### Exemple 6

### Préparation du complexe 8 de formule [Tb.(1-4H).H₂O]Na

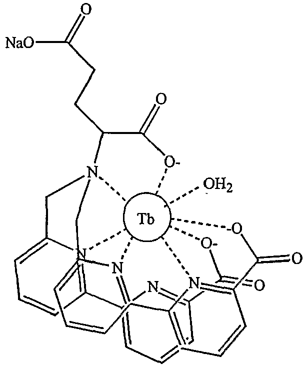

Dans un ballon de 250 mL équipé d'un réfrigérant, 40 mg (59 µmol) de composé **1**.3HCl sont dissous dans un mélange de 30 mL de MeOH et de 30 mL d'eau. A cette solution sont ajoutés 25 mg (67 µmol) de TbCl₃.6H₂O dissous dans 5 mL de MeOH et 5 mL d'eau. La solution est chauffée à 70°C pendant une heure. Après refroidissement, le pH de la solution est porté à 7,2 avec une solution à 1% de NaOH dans l'eau. La solution est concentrée à l'évaporateur rotatif jusqu'à apparition d'un léger trouble, puis on ajoute du THF jusqu'à la formation d'un important précipité. Un solide jaune pâle est isolé par centrifugation, puis séché sous vide. On obtient 46 mg (56 µmol) de complexe **8** (correspondant à un rendement de 95%) dont les caractéristiques sont les suivantes :
Analyses calculées pour C₂₉H₂₁NaN₅O₈Tb.4H₂O : C 42,40, H 3,56, N 8,53. Trouvées : C 42,28, H 3,31, N 8,38.
FAB⁻/MS _{:} 668,2 ([**8**-H₂O-CH₂COONa]⁻, 100%), 726,2 ([**8**-H₂O-Na]⁻, 30%).
IR (pastille de KBr, cm⁻¹) : 3428, 1592, 1574, 1466, 1416, 1387.

### Propriétés photo-physiques dans l'eau:

Absorption, λₘₐₓ [nm] (εₘₐₓ [M⁻¹.cm⁻¹]): 320 (épaulement), 308 (20800), 277 (8900), 267 (10400), 253 (15000).
Emission : caractéristique des composés du terbium avec des bandes fines à 487, 543, 583 et 621 nm. Temps de vie de l'état excité : 1,48 ms. Rendement quantique (référence sulphate de quinine dans H₂SO₄ 1N) : 31%. Temps de vie de l'état excité dans l'eau lourde : 2,53 ms. Rendement quantique dans l'eau lourde : 53%.

### Exemple 7

### Préparation du complexe 9 de formule

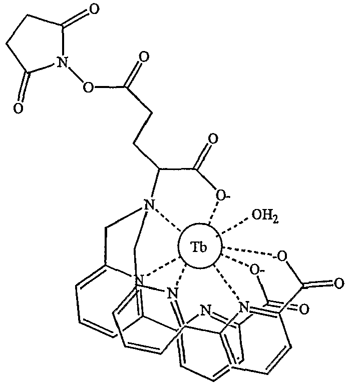

Dans un ballon de 10 mL, 50 mg (61 µmol) de complexe **8** sont mis en suspension dans 5 mL de DMSO. A cette solution on ajoute 9 mg (78 µmol) de N-hydroxysuccinimide et 13 mg (68 µmol) de sels d'hydrochlorure d'éthyle-N,N-diméthyle-3-aminopropyle-carbodiimide (EDCI.HCl). La solution est agitée à température ambiante pendant 138 heures durant lesquelles le complexe **8** se dissout, puis un précipité blanc se forme. Le solide est isolé par centrifugation, lavé au THF et séché sous vide. L'addition de THF aux eaux-mères provoque la formation d'un précipité supplémentaire, que l'on récupère par centrifugation. On obtient en tout 49 mg (55 µmol) de complexe **9** (correspondant à un rendement de 90%) dont les caractéristiques sont les suivantes :
Analyses calculées pour C₃₃H₂₅N₆O₁₀Tb.₄H₂O : C 44,21, H 3,71, N 9,29. Trouvées : C 44,01, H 3,42, N 9,29.
FAB⁺/MS : 726,2 ([**9**-H₂O-C₄H₄NO₂]⁺, 15%), 825,5 ([**9**-H₂O+H]⁺, 100%).
IR (pastille de KBr, cm⁻¹) : 3433, 1741, 1624, 1594, 1574, 1464, 1419, 1375.

### Propriétés photo-physiques dans l'eau

Absorption, λₘₐₓ [nm] (εₘₐₓ [M⁻¹.cm⁻¹]) : 308 (18700), 276, 267, 253.
Emission : caractéristique des composés du terbium avec des bandes fines à 487, 543, 583 et 621 nm. Temps de vie de l'état excité : 1,50 ms. Rendement quantique (référence sulphate de quinine dans H₂SO₄ 1N) : 34%. Temps de vie de l'état excité dans l'eau lourde : 2,42 ms. Rendement quantique dans l'eau lourde : 62%.

### Exemple 8

### Marquage de l'albumine sérique de boeuf ASB par le complexe 9 et mise en évidence par microscopie de luminescence en temps résolu.

Le marquage de l'albumine sérique de boeuf a été effectué selon la méthode décrite dans l'exemple **5** en remplaceant le complexe **6** par le complexe **9.**

### Détermination du rapport molaire marqueurs/BSA

Le rapport molaire marqueurs/BSA (nombre de complexes **9** liés de façon covalente à l'ASB) est déterminé par absorption différentielle à 308 nm. Les coefficients d'absorption molaire de l'ASB native et de l'ASB marquée sont mesurés à 308 nm. La différence de ces deux valeurs est divisée par le coefficient d'absorption molaire du complexe **9** à 308 nm, pour donner un rapport molaire marqueurs/BSA de 6/1 dans la protéine marquée.

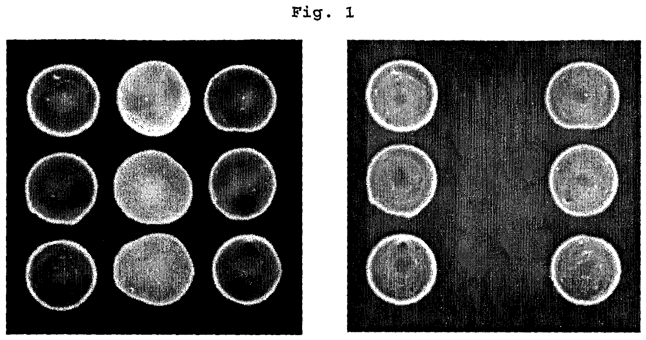

La figure 1 représente des goutelettes d'environ 750 microns de diamètre contenant l'ASB marquée par le composé **9** (colonnes de gauche et de droite sur chaque image) et un anticorps marqué par de la fluorescéine (colonne du milieu sur chaque image) servant de référence (Immunoglobuline de lapin marquée à la fluorescéine produit par Dako-Immunoglobuline sous le code produit F-123). L'image obtenue par microscopie de fluorescence conventionnelle (gauche) met en évidence la fluorescence des deux composés. L'image obtenue par microscopie de luminescence en temps résolu (délai = 0,5 ms, temps d'intégration = 5,0 ms) montre la disparition de la fluorescence du composé de référence alors que la luminescence de l'ASB marquée perdure.

### Exemple 9

### Préparation du complexe 10 de formule [Gd.(1-4H).H₂O]Na

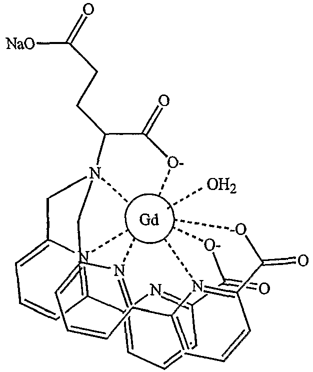

Dans un ballon de 100 mL équipé d'un réfrigérant, 30 mg (44 µmol) de composé **1**.3HCl sont dissous dans un mélange de 25 mL de MeOH et de 25 mL d'eau. A cette solution on ajoute 19 mg (51 µmol) de GdCl₃.6H₂O dissous dans 5 mL de MeOH et 5 mL d'eau. La solution est chauffée à 70°C pendant une heure. Après refroidissement le pH de la solution est remonté à 7,5 avec une solution à 0,5% de NaOH dans l'eau. La solution est concentrée à l'évaporateur rotatif jusqu'à apparition d'un léger trouble puis on ajoute du THF jusqu'à la formation d'un important précipité. Le solide jaune pâle est isolé par centrifugation puis séché sous vide pour donner 30 mg (37 µmol) de complexe **10** (correspondant à un rendement de 85%) dont les caractéristiques sont les suivantes :
Analyses calculées pour C₂₉H₂₁GdNaN₅O₈.3H₂O : C 43,44, H 3,39, N 8,73. Trouvées : C 43,35, H 3,17, N 8,55.
FAB⁻/MS : 667,2 ([**10**-H₂O-CH₂COONa]⁻, 100%), 725,2 ([**10**-H₂O-Na]⁻, 45%).
IR (pastille de KBr, cm⁻¹) : 3422, 1637, 1592, 1459, 1419, 1385.

### Exemple 10

### Préparation du complexe 11 de formule

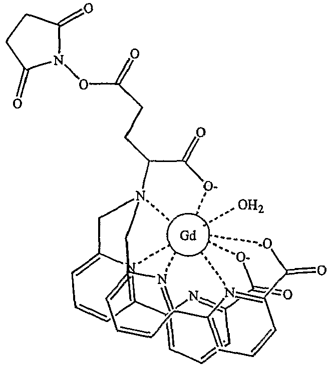

Dans un ballon de 10 mL, 50 mg (62 µmol) de composé **10** sont mis en suspension dans 5 mL de DMSO. A cette solution on ajoute 9 mg (78 µmol) de N-hydroxysuccinimide et 15 mg (78 µmol) de sels d'hydrochlorure d'éthyle-N,N-diméthyle-3-aminopropyle-carbodiimide (EDCI.HCl). La solution est agitée à température ambiante pendant 48 heures durant lesquelles le complexe **10** se dissout, puis un précipité blanc se forme. Le solide est isolé par centrifugation, lavé au THF et séché sous vide. L'addition de THF aux eaux-mères provoque la formation de précipité supplémentaire, que l'on récupère par centrifugation. En tout, on obtient 45 mg (51 µmol) de complexe **11** (correspondant à un rendement de 82%) dont les caractéristiques sont les suivantes :
Analyses calculées pour C₃₃H₂₅GdN₆O₁₀.3H₂O : C 45,20, H 3,56, N 9,37. Trouvées : C 45,02, H 3,18, N 9,21.
FAB⁺/MS: 726,5 ([**11**-H₂O-C₄H₄NO₂+2H]⁺, 20%), 824,2 ([**11-**H₂O+H]⁺, 100%).
IR (pastille de KBr, cm⁻¹) : 3435, 1741, 1623, 1573, 1465, 1420, 1376.

### Exemple 11

### Préparation du composé 12

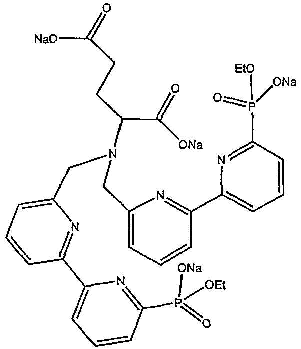

Ce composé est obtenu en deux étapes à partir du composé **3** selon le schéma synthétique suivant :

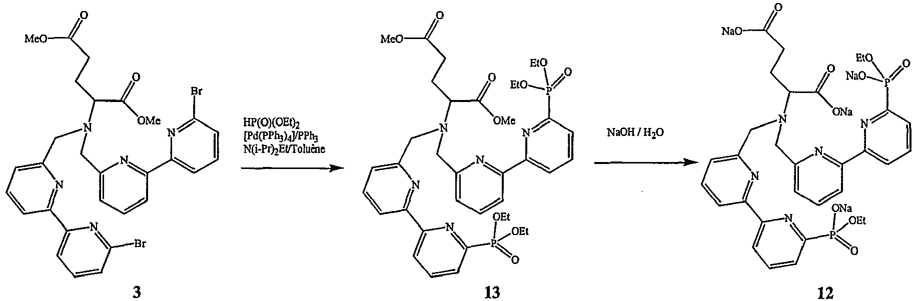

### Préparation du composé 13

Dans un tube de Schlenk sous atmosphère d'argon, on introduit 200 mg (0,30 mmol) de composé **3**, 90 µL (0,70 mmol) de diéthylphosphite, 78 mg (0,30 mmol) de PPh₃ et 300 µL de diisopropyléthylamine fraîchement distillée dans 10 mL de toluène. La solution est dégazée à l'argon pendant 20 minutes. On ajoute 34 mg (0,03 mmol) de Pd(PPh₃)₄ et la solution est chauffée à 100°C pendant 16 heures. On ajoute 40 µL (0,31 mmol) de diéthylphosphite et 34 mg (0,03 mmol) de Pd(PPh₃)₄ et la solution est à nouveau chauffée à 100°C pendant 16 heures. La solution est évaporée à sec. Le résidu solide est purifié par chromatographie flash sur silice (φ=3 cm, h=15 cm) avec un mélange de CH₂Cl₂/MeOH (99/1 à 95/5) comme éluant. Les fractions pures sont évaporées, solubilisées dans 30 mL de CH₂Cl₂ et lavées avec 10 mL d'eau. La phase organique est séchée sur MgSO₄, filtrée et évaporée. On obtient 72 mg (0,09 mmol) de composé **13** (correspondant à un rendement de 31%) sous forme d'une huile qui présente les caractéristiques suivantes :

*R_{f}* = 0,56, SiO₂, CH₂Cl₂/MeOH (90/10).

¹H-RMN (CDCl₃, 200 MHz) : δ 1,35 (t, 12H, ³J=7,0 Hz), 2,02-2,22 (m, 2H), 2,37-2,71 (m, 2H), 3,47 (s, 3H), 3,54-3,61 (m, 1H), 3,75 (s, 3H), 4,01-4,17 (m, 4H), 4,18-4,36 (m, 8H), 7,47 (d, 2H, ³J=7,5 Hz), 7,73 (t, 2H, ³J=8,0 Hz), 7,81-7,97 (m, 4H), 8,32 (d, 2H, ³J=7,5 Hz), 8,59 (dt, 2H, ³J_{H-H}=7,0 Hz, ³J_{H-P}=⁴J_{H-H}=2,0 Hz).
¹³C-RMN (CDCl₃, 50 MHz) : δ 16,3, 16,4, 24,7, 30,3, 51,4, 57,1, 61,9, 63,0, 63,1, 119,6, 123,2 (2), 123,4, 127,4, 127,9, 136,7, 137,0, 137,2, 149,0, 153,5, 154,5, 156,5, 156,9, 159,0, 173,1, 173,4.
³¹P-RMN (CDCl₃, 162 MHz) : δ 11,73.

### Préparation du composé 12

Dans un ballon de 50 mL équipé d'un réfrigérant, 51 mg (65 µmol) de composé **13** sont dissous dans 6 mL de solution de NaOH 0,05 N dans l'eau. Le mélange est chauffé à 100°C pendant 19 heures. Après refroidissement la phase aqueuse est extraite avec 4 portions de 5 mL de CH₂Cl₂ puis évaporée à sec. Le produit précipite dans un mélange H₂O/THF. On obtient 45 mg (51 µmol) de composé **12** (correspondant à un rendement de 79%) sous forme de poudre de couleur crème dont les caractéristiques sont les suivantes :
¹H-RMN (D₂O/^{t}BuOH, 300 MHz) : δ 1,18 (t, 6H, ³J=7,0 Hz), 2,06-2,27 (m, 2H), 2,37-2,58 (m, 2H), 3,50 (t, 3H, ³J=7,5 Hz), 3,86-3,99 (m, 4H), 4,02-4,24 (m, 4H), 7,48 (d, 2H, ³J=7,0 Hz), 7,59-7,81 (m, 10H).
¹³C-RMN (D₂O/^{t}BuOH, 75 MHz) : δ 16,4, 16,5, 27,8, 35,6, 59,8, 62,4, 62,5, 71,6, 121,2, 124,0, 124,1, 125,7, 127,1, 127,4, 138,0, 138,2, 138,5, 154,6, 155,0, 156,3, 156,6, 157,8, 160,6, 181,1, 183,6.
³¹P-RMN (D₂O 162 MHz) : δ 10,17
Analyses calculées pour C₃₁H₃₁N₅Na₄O₁₀P₂.5H₂O : C 42, 43, H 4,71, N 7,98. Trouvées : C 42,35, H 4,55, N 7,78.
FAB⁺/MS : 764,2 ([**12**-Na]⁺, 10%).

### Exemple 12

### Préparation du complexe 14 de formule

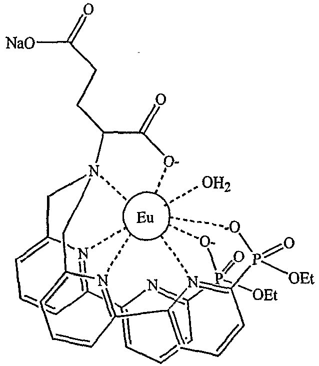

Dans un ballon de 50 mL équipé d'un réfrigérant, 19 mg (22 µmol) de composé **12** sont dissous dans 35 mL d'eau. Le pH est ajusté à 3,1 avec une solution de HCl dilué. A cette solution on ajoute 9 mg (25 µmol) de EuCl₃.6H₂O dissous dans 5 mL d'eau. La solution est chauffée à 80°C pendant une heure. Après refroidissement la solution est filtrée sur célite et le pH est remonté à 7,1 avec une solution à 0,5% de NaOH dans l'eau. La solution est évaporée à sec, le produit précipite dans un mélange H₂O/THF. Le solide jaune pâle est isolé par centrifugation puis séché sous vide, et l'on obtient 9 mg (10 µmol) de complexe **14** (correspondant à un rendement de 47%) dont les caractéristiques sont les suivantes :
FAB⁺/MS : 848,2 ([**14**-H₂O-Na]⁺, 35%).

### Exemple 13

### Préparation du composé 15

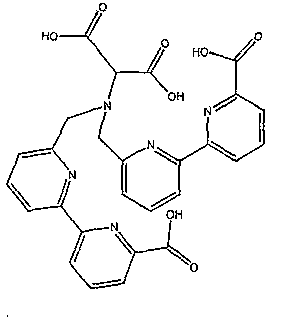

Ce composé est obtenu en trois étapes selon le schéma synthétique suivant:

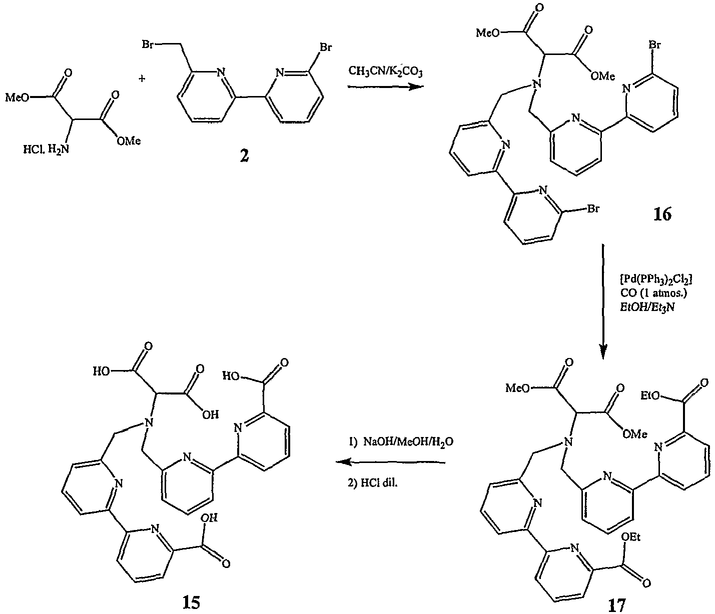

### Préparation du composé 16

Dans un ballon de Schlenk de 500 mL sous atmosphère d'argon, on introduit 450 mg (2,13 mmol) de sels d'hydhrochlorure de diéthylaminomalonate et 1,18 g (8,54 mmol) de K₂CO₃ dans 150 mL d'acétonitrile fraîchement distillée. La solution est chauffée à 80°C pendant une heure. On ajoute 1,46 g (4,45 mmol) de composé **2** et on chauffe pendant 21 heures à 80°C. La solution est évaporée à sec et le résidu est redissous avec 100 mL de CH₂Cl₂ et 20 mL d'eau. La phase aqueuse est lavée avec deux portions de 20 mL de CH₂Cl₂ et la somme des phases organiques est séchée sur MgSO₄, filtrée, puis évaporée à sec. Le résidu solide est purifié par chromatographie flash sur silice (φ=4 cm, h=14 cm) avec un mélange de CH₂Cl₂/MeOH (100/0 à 99/1) comme éluant. On obtient 794 mg (1,19 mmol) de composé **16** (correspondant à un rendement de 56%) sous forme de poudre jaune pâle qui présente les caractéristiques suivantes :

*R_{f}* = 0,57, SiO₂, CH₂Cl₂/MeOH (97/3).

¹H-RMN (CDCl₃, 200 MHz) : δ 1,26 (t, 6H, ³J=7,0 Hz), 4,22 (s, 4H), 4,23 (q, 4H, ³J=7,0 Hz), 4,47 (s, 1H), 7,43 (dd, 2H, ³J=7,5 Hz, ⁴J=0,5 Hz), 7,60 (t, 2H, ³J=7,5 Hz), 7,62 (d, 2H, ³J=7,5 Hz), 7,75 (t, 2H, ³J=8,0 Hz), 8,22 (dd, 2H, ³J=7,5 Hz, ⁴J=1,0 Hz), 8,37 (dd, 2H, ³J=7,5 Hz, ⁴J=1,0 Hz).
¹³C-RMN (CDCl₃, 50 MHz) : δ 14,1, 58,0, 61,4, 67,1, 119,7, 123,4, 127,7, 137,4, 139,0, 141,4, 153,5, 157,4, 158,9, 168,1.
Analyses calculées pour C₂₉H₂₇Br₂N₅O₄ : C 52,04, H 4,07, N 10,46. Trouvées : C 51,93, H 3,93, N 10,31.
FAB⁺/MS : 670,2 (100%), 672,2 (50%), [**16**+H]⁺.

### Préparation du composé 17

Dans un ballon bicol de 250 mL on introduit 778 mg (1,16 mmol) de composé **16** et 82 mg (0,12 mmol) de [Pd(PPh₃)₂Cl₂] dans 75 mL d'éthanol et 75 mL de triéthyl-amine. La solution est chauffée à 70°C pendant 16 heures en faisant barboter un flux de CO. La solution est évaporée à sec, le solide obtenu est redissous dans 75 mL de CH₂Cl₂, filtré sur célite, puis la phase organique est lavée avec 15 mL d'eau. La phase aqueuse est extraite avec deux portions de 20 mL de CH₂Cl₂ et l'ensemble des phases organiques est séché sur MgSO₄, filtré puis évaporé à sec. Le résidu est purifié par chromatographie flash sur silice (φ=3 cm, h=16 cm) avec un mélange de CH₂Cl₂/MeOH (99,5/0,5 à 90/10) comme éluant. Les fractions contenant le composé **17** avec de l'oxyde de triphénylphosphine sont dissoutes dans 40 mL de CH₂Cl₂ et extraites avec quatre portions de HCl 3N. Les phases aqueuses combinées sont neutralisées avec NaOH puis extraites avec trois portions de 30 mL de CH₂Cl₂. L'ensemble des phases organiques est séché sur MgSO₄, filtré puis évaporé à sec. On obtient 522 mg (0,80 mmol) de composé **17** sous forme d'une huile incolore (correspondant à un rendement de 68%), qui présente les caractéristiques suivantes :

*R_{f}* = 0,55, SiO₂, CH₂Cl₂/MeOH (90/10).

¹H-RMN (CDCl₃, 200 MHz) : δ 1,26 (t, 6H, ³J=7,0 Hz), 1,45 (t, 6H, ³J=7,0 Hz), 4,23 (q, 4H, ³J=7,0 Hz), 4,24 (s, 4H), 4,47 (q, 4H, ³J=7,0 Hz), 4,48 (s, 1H), 7,64 (dd, 2H, ³J=7,5 Hz, ⁴J=0,5 Hz), 7,80 (t, 2H, ³J=8,0 Hz), 7,91 (t, 2H, ³J=7,5 Hz), 8,09 (dd, 2H, ³J=7,5 Hz, ⁴J=1,0 Hz), 8,40 (dd, 2H, ³J=7,5 Hz, ⁴J=0,5 Hz), 8,62 (dd, 2H, ³J=8,0 Hz, ⁴J=1,5 Hz).
¹³C-RMN (CDCl₃, 50 MHz) : δ 14,1, 14,3, 58,0, 61,4, 61,8, 67,1, 120,0, 123,4, 124,2, 124,7, 137,5, 137,7, 147,7, 154,4, 156,5, 158,8, 165,3, 168,2.
Analyses calculées pour C₃₅H₃₇N₅O_{8 :} C 64,11, H 5,69, N 10,68. Trouvées : C 63,81, H 5,43, N 10,43.
FAB⁺/MS : 496,2 (35%), 656,1 ([**17**+H]⁺, 100%).

### Préparation du composé 15

Dans un ballon de 50 mL équipé d'un réfrigérant, 103 mg (0,16 mmol) de composé **17** et 50 mg (1,25 mmol) de NaOH sont dissous dans un mélange de 10 mL de MeOH et 5 mL d'eau. Le mélange est chauffé à 70°C pendant 5 heures. La solution est évaporée à sec et le solide est dissous dans 8 mL d'eau sur lesquels on ajoute lentement à 0°C une solution de HCl 1N jusqu'à ce que le produit précipite massivement (pH=4-5). Le précipité est isolé par centrifugation et séché sous vide. On obtient 59 mg (0,08 mmol) de chlorhydrate hydraté 1**5.**3HCl (correspondant à un rendement de 53%) sous forme de poudre blanche dont les caractéristiques sont les suivantes :
¹H-RMN (NaOD/^{t}BuOH, 300 MHz) : δ 3,75 (s, 4H), 4,04 (s, 1H), 6,84 (d, 2H, ³J=7,5 Hz), 7,15-7,26 (m, 4H), 7,32 (d, 2H, ³J=7,5 Hz), 7,42 (t, 2H, ³J=7,5 Hz), 7,56 (d, 2H, ³J=7,5 Hz).
¹³C-RMN (NaOD/^{t}BuOH, 75 MHz) : δ 60,3, 79,4, 119,9, 122,9, 124,1, 124,4, 138,2, 138,6, 152,8, 153,7, 154,0, 158,7, 168,6, 172,3, 177,3.
Analyses calculées pour C₂₇H₂₁N₅O₈.3HCl.3H₂O: C 45,87, H 4,28, N 9,91. Trouvées : C 45,75, H 4,09, N 9,78.
FAB⁺/MS : 544,2 ([15+H]⁺, 20%).

### Exemple 14

### Préparation du complexe 18 de formule

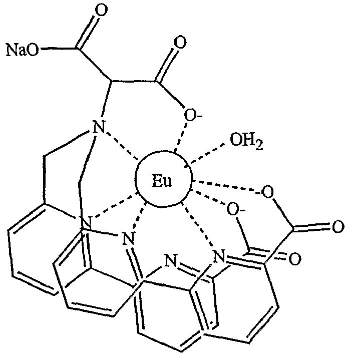

Dans un ballon de 10 mL, 15 mg de **15**.3HCl.3H₂O (21 µmol) sont dispersés dans un mélange de 10 mL de MeOH et de 10 mL d'eau. A cette solution est ajouté 10 mg (27 µmol) de EuCl₃.6H₂O dissous dans 5 mL de MeOH et 5 mL d'eau. La solution est chauffée à 70°C pendant une heure. Après refroidissement le pH de la solution est remonté à 7,3 avec une solution à 0,5% de NaOH dans l'eau. La solution est concentrée à l'évaporateur rotatif jusqu'à apparition d'un précipité. Le solide blanc est isolé par centrifugation puis séché sous vide pour donner 14 mg (19 µmol) de composé **18** (correspondant à un rendement de 90%) dont les caractéristiques sont les suivantes :
FAB⁻/MS : 692,3 ([**18**-H₂O-Na)]⁻, 100%).
IR (pastille de KBr, cm⁻¹) : 3442, 1626, 1588, 1460, 1411, 1373.

### Exemple 15

### Préparation du complexe 19 de formule :

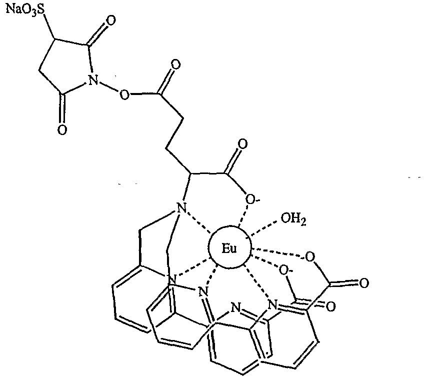

Dans un ballon de 10 mL, 18 mg (22 µmol) de complexe **5** sont mis en suspension dans 5 mL de DMSO. A cette solution on ajoute 6 mg (26 µmol) de sel monosodique de N-hydroxy-succinimide-3-acide sulfonique hydraté et 5 mg (26 µmol) de chlorhydrate d'éthyle-N,N-diméthyle-3-aminopropyle-carbodiimide (EDCI.HCl). La solution est agitée à température ambiante pendant 46 heures durant lesquelles le complexe **5** se dissout. L'ajout de THF à la solution provoque la formation d'un précipité que l'on récupère par centrifugation. On obtient 15 mg (15 µmol) de complexe **19** (correspondant à un rendement de 68%) sous forme de poudre blanche.

### Exemple 16

### Préparation du complexe 20 de formule :

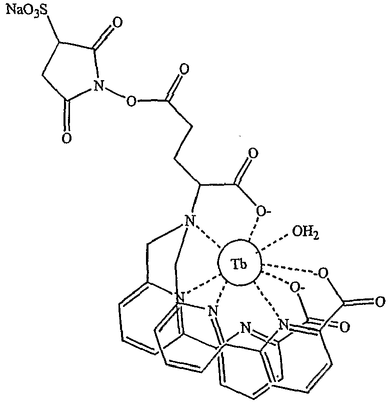

Dans un ballon de 50 mL, 45 mg (55 µmol) de complexe **8** sont mis en suspension dans 10 mL de DMSO. A cette solution on ajoute 14 mg (60 µmol) de sel monosodique de N-hydroxy-succinimide-3-acide sulfonique hydraté et 12 mg (63 µmol) de chlorhydrate d'éthyle-N,N-diméthyle-3-aminopropyle-carbodiimide (EDCI.HCl). La solution est agitée à température ambiante pendant 92 heures durant lesquelles le complexe **8** se dissout. L'ajout de THF à la solution provoque la formation d'un précipité que l'on récupère par centrifugation. On obtient 45 mg (44 µmol) de complexe **20** (correspondant à un rendement de 81%) sous forme de poudre jaune dont les caractéristiques sont les suivantes :
Analyses calculées pour C₃₃H₂₄N₆NaO₁₃STb.5H₂O : C 38,99, H 3,37, N 8,27. Trouvées : C 39,20, H 3,56, N 8,39.
FAB⁺/MS : 682,2 ([**20**-H₂O-C₅H₃NNaO₇S]⁺, 95%), 727,2 ([**20**-H₂O-C₄H₃NNaO₅S+H]⁺, 55%).

### Exemple 17

### Préparation du complexe 21 de formule :

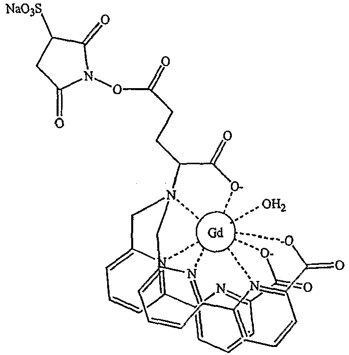

Dans un ballon de 10 mL, 19 mg (24 µmol) de complexe **10** sont mis en suspension dans 5 mL de DMSO. A cette solution on ajoute 7 mg (30 µmol) de sel monosodique de N-hydroxysuccinimide-3-acide sulfonique hydraté et 5 mg (26 µmol) de chlorhydrate d'éthyle-N,N-diméthyle-3-aminopropyle-carbodiimide (EDCI.HCl). La solution est agitée à température ambiante pendant 24 heures durant lesquelles le complexe **10** se dissout. L'ajout de THF à la solution provoque la formation d'un précipité que l'on récupère par centrifugation. On obtient 19 mg (19 µmol) de complexe **21** (correspondant à un rendement de 80%) sous forme de poudre jaune dont les caractéristiques sont les suivantes :
FAB⁺/MS : 681,2 ([**21**-H₂O-C₅H₃NNaO₇S]⁺, 100%), 726,3 ([**21**-H₂O-C₄H₃NNaO₅S+H]⁺, 40%).

### Exemple 18

### Préparation du compose 25

Le compose **25** est obtenu en quatre étapes selon le schéma suivant:

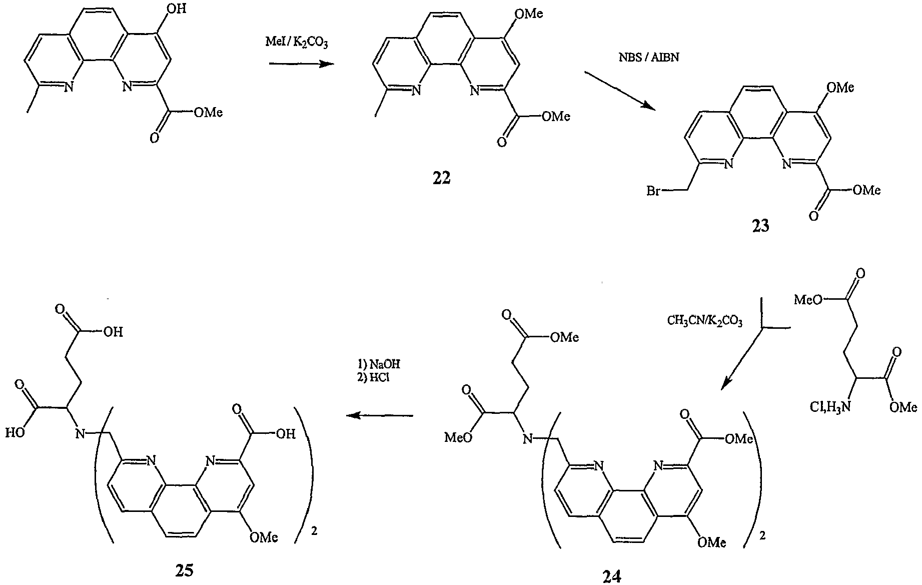

### Préparation du composé 22

Dans un tube de Schlenk sous atmosphère d'argon, on introduit 2,04 g (7,6 mmol) de 7-hydroxy-9-carbométhoxy-2-méthyl-phénanthroline (obtenu selon Heindel, N. et al, J. Heterocycl. Chem. 1968, 5, 869), 2,11 g (15,2 mmol) de K₂CO₃ et 950 µL (15,3 mmol) d'iodure de méthyle dans 60 mL d'acétonitrile fraîchement distillée sur P₂O₅. La solution est chauffée à 80°C pendant 19 heures. La solution est évaporée à sec, le résidu est dissous dans 100 mL de CH₂Cl₂ et 15 mL d'eau. La phase aqueuse est extraite avec 4 portions de 15 mL de CH₂Cl₂ et la somme des phases organiques est séchée sur MgSO₄, filtrée, puis évaporée à sec. Le résidu est purifié par chromatographie sur alumine (φ=5 cm, h=12 cm) avec un mélange de CH₂Cl₂/MeOH (99/1) comme éluant. On obtient 2,05 g (7,3 mmol) de composé **22** (correspondant à un rendement de 95%) sous forme de poudre jaune qui présente les caractéristiques suivantes :

*R_{f}* = 0,54, Al₂O₃, CH₂Cl_{2/}MeOH (98/2).

¹H-RMN (CDCl₃, 200 MHz) : δ 2,91 (s, 3H), 4,06 (s, 3H), 4,12 (s, 3H), 7,47 (d, 1H, ³J=8,5 Hz), 7,77 (d, 1H, ³J=9,0 Hz), 7,83 (s, 1H), 8,08 (d, 1H, ³J=7,5 Hz), 8,12 (d, 1H, ³J=9,0 Hz).
¹³C-RMN (CDCl₃, 50 MHz) : δ 25,8, 52,8, 56,2, 102,9, 118,7, 122,2, 123,9, 126,9, 127,4, 136,0, 145,2, 146,1, 148,6, 160,1, 163,2, 166,5.
Analyses calculées pour C₁₆H₁₄N₂O₃ : C 68,07, H 5,00, N 9,92. Trouvées : C 67,92, H 4,93, N 9,78.
FAB⁺/MS : 283,2 ([**22**+H]⁺, 100%).

### Préparation du composé 23

Dans un ballon de 250 mL, on introduit 1 g (3,5 mmol) de composé **22,** 630 mg (3,5 mmol) de N-bromosuccinimide et 30 mg (0,2 mmol) d'azo-bis-isobutyronitrile (AIBN) dans 10 mL de benzène. La solution est irradiée pendant 30 minutes avec une lampe halogène standard de 100 W. Le solvant est évaporé sous pression réduite et le résidu est purifié par chromatographie sur alumine contenant 10% d'eau avec un mélange de CH₂Cl₂/hexane (50/50) comme éluant. On obtient 468 mg (1,3 mmol) de composé **23** (correspondant à un rendement de 37%) sous forme de poudre grise qui présente les caractéristiques suivantes :

*R_{f}* = 0,71, Al₂O₃, CH₂Cl₂/MeOH (98/2) .

¹H-RMN (CDCl₃, 200 MHz) : δ 4,06 (s, 3H), 4,12 (s, 3H), 4,93 (s, 2H), 7,77 (d, 1H, ³J=9,0 Hz), 7,83 (s, 1H), 7,87 (d, 1H, ³J=8,5 Hz), 8,17 (d, 1H, ³J=9,0 Hz), 8,21 (d, 1H, ³J=8,5 Hz). ¹³C-RMN (CDCl₃, 50 MHz) : δ 34,6, 53,0, 56,3, 103,3, 120,2, 122,4, 123,7, 127,0, 128,1, 137,1, 144,5, 145,9, 148,9, 157,6, 163,3, 166,2.
Analyses calculées pour C₁₆H₁₃BrN₂O₃ : C 53,21, H 3,63, N 7,76. Trouvées : C 52,94, H 3,26, N 7,51.
FAB⁺/MS : 281,2 ([**23**-Br]⁺, 30%), 361,2 (100%), 363,2 (100%), [**23**+H]⁺.

### Préparation du composé 24

Dans un tube de Schlenk sous atmosphère d'argon, on introduit 96 mg (0,45 mmol) de chlorhydrate du diméthyle ester de l'acide DL-glutamique et 250 mg (1,81 mmol) de K₂CO₃ dans 15 mL d'acétonitrile fraîchement distillée sur P₂O₅. La solution est chauffée à 80°C pendant 10 minutes. On ajoute 360 g (1 mmol) de composé **23** et on chauffe pendant 18 heures à 80°C. On ajoute une nouvelle portion de composé **23** (52 mg, 0,14 mmol) et on chauffe pendant 24 heures à 80°C. La solution est évaporée à sec, le résidu est dissous dans 30 mL de CH₂Cl₂ et 10 mL d'eau. La phase aqueuse est extraite avec 4 portions de 30 mL de CH₂Cl₂ et la somme des phases organiques est séchée sur MgSO₄, filtrée, puis évaporée à sec. Le résidu est purifié par chromatographie sur alumine contenant 10% d'eau avec un mélange de CH₂Cl₂/MeOH (100/0 à 99,3/0,7) comme éluant. On obtient 46 mg (0,06 mmol) de composé **24** (correspondant à un rendement de 37%) qui présente les caractéristiques suivantes :

*R_{f}* = 0,16, Al₂O₃, CH₂Cl₂/MeOH (98/2).

¹H-RMN (CDCl₃, 200 MHz) : δ 2,17-2,28 (m, 2H), 2,61 (t, 2H, ³J=7,5 Hz), 3,44 (s, 3H), 3,71 (t, 1H, ³J=7,5 Hz), 3,83 (s, 3H), 4,06-4,19 (m, 12H), 4,45-4,70 (m, 4H), 7,82 (d, 2H, ³J=9,0 Hz), 7,86 (s, 2H), 8,13 (d, 2H, ³J=8,0 Hz), 8,18 (d, 2H, ³J=9,0 Hz), 8,24 (d, 2H, ³J=8,5 Hz).

### Préparation du composé 25.3HCl

Dans un ballon de 50 mL, 46 mg (0,06 mmol) de **24** et 10 mg (0,25 mmol) de NaOH sont dissous dans un mélange de 9 mL de MeOH et 3 mL d'eau. Le mélange est chauffé à 75°C pendant 21 heures. Les solvants sont évaporés sous pression réduite, le solide est dissous dans 5 mL d'eau et la solution obtenue est filtrée sur célite. Le milieu est acidifié avec une solution diluée d'acide chlorhydrique et la solution est évaporée à sec. Le résidu est lavé avec 2 portions de 2 mL d'eau. On obtient 19 mg (0,02 mmol) de chlorhydrate 2**5.**3HCl (correspondant à un rendement de 39%) sous forme de poudre jaune-orange dont les caractéristiques sont les suivantes :
¹H-RMN (CD₃OD, 200 MHz) : δ 2,38-2,52 (m, 2H), 2,84 (t, 2H, ³J=7,0 Hz), 4,17 (t, 1H, ³J=7,5 Hz), 4,35 (s, 6H), 4,81-4,85 (m, 4H), 7,87 (d, 2H, ³J=9,5 Hz), 7,89 (s, 2H), 8,16 (d, 2H, ³J=9,0 Hz), 8,18 (d, 2H, ³J=8,5 Hz), 8,65 (d, 2H, ³J=9,0 Hz).

### Exemple 19

### Préparation du composé 26 de formule [Eu.(25-4H).H₂O]Na

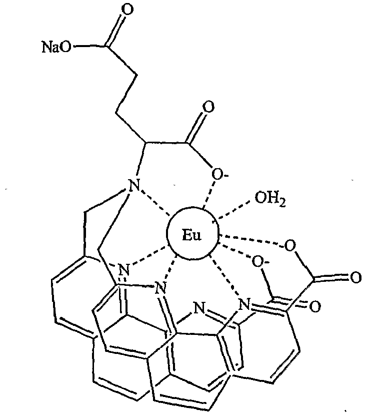

Dans un ballon de 50 mL, 19 mg de **25**.3HCl (24 µmol) sont dissous dans un mélange de 15 mL de MeOH et de 15 mL d'eau. A cette solution est ajouté 10 mg (27 µmol) de EuCl₃.6H₂O dissous dans 2,5 mL de MeOH et 2,5 mL d'eau. La solution est chauffée à 70°C pendant une heure. Après refroidissement le pH de la solution est remonté à 7,0 avec une solution à 0,5% de NaOH dans l'eau. La solution est concentrée à l'évaporateur rotatif jusqu'à apparition d'un léger trouble, puis on ajoute du THF jusqu'à la formation d'un précipité. Le solide jaune est isolé par centrifugation puis séché sous vide pour donner 7 mg (8 µmol) de composé **26** (correspondant à un rendement de 33%) dont les caractéristiques sont les suivantes :
FAB⁻/MS : 791,2 (30%), 828,2 ([**26**-H₂O-Na)]⁻, 50%).

### Exemple 20

### Marquage d'un anticorps anti-digoxigénine par le complexe 9 et caractérisation par spectrométrie de masse.

0,5 mg de complexe **9** sont ajoutés à une solution d'anticorps anti-digoxigénine contenant 1,0 mg d'anticorps dissous dans 500 µL de solution tampon (tampon borate 50 mM, pH = 7.0), correspondant à un rapport **9**/anticorps de 30:1, La solution est agitée à température ambiante pendant 24 heures, puis l'anticorps marqué est purifié selon la procédure décrite dans l'exemple **5** et stocké à 4°C.

La caractérisation par spectrométrie de masse MALDI-TOF est effectuée selon la procédure décrite dans l'exemple **5**, conduisant à une masse de 49220 Da pour l'anticorps marqué (47880 Da pour l'anticorps libre), soit un taux de greffage de 2,5.

## Revendications

1. Composé répondant à la formule (I) : 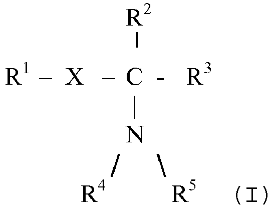 sous forme cationique, zwitterionique ou anionique,
dans laquelle
- R¹ est un groupe choisi parmi les groupes carboxyle, ester N-succinimidique et ester sulfosuccinimidique ;
- X représente une simple liaison ou un groupement alkylène ayant 2 atomes de carbone ;
- R² est un groupement anionique à pH neutre A² choisi parmi les groupes CO₂H, SO₃H, P(O)(OR)OH, P(O)R(OH) et P(O)(OH)₂ dans lesquels R est un groupe alkyle ou un groupe aryle ;
- R³ représente H ;
- le substituant R⁴ comprend un groupement 2,2'-bipyridyle portant un groupe A⁴ choisi parmi un carboxyle, un monoalkylphosphonate, un monoarylphosphonate et un phosphonyle, ou un groupement phénanthrolinyle portant un groupe A⁴ carboxyle, le substituant R⁴ correspondant à l'une des formules (II), (III), (IV) et (V) suivantes : 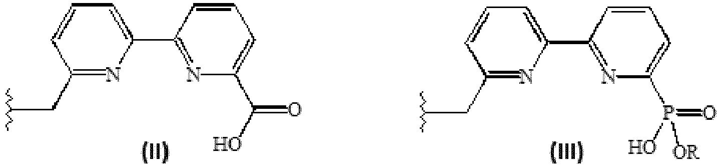 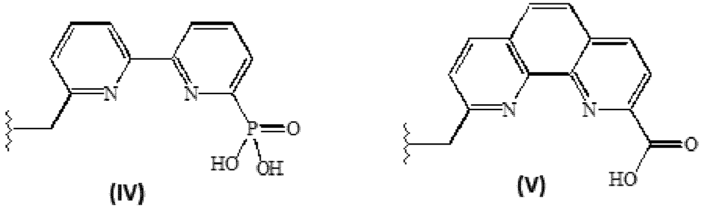 R étant aryle ou alkyle ;
- le substituant R⁵ est identique à R⁴, chacun des substituants R⁴ et R⁵ étant un substituant monovalent, les substituants R⁴ et R⁵ ne formant pas ensemble un groupe divalent, et
- dans les substituants R² , R⁴ et R⁵, R est un groupe alkyle en C₁ à C₃ ou un groupe aryle en C₅ à C₉.

2. Composé répondant à la formule (I): 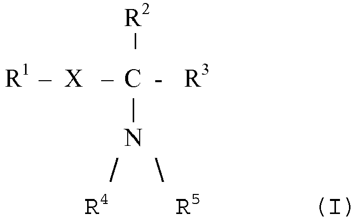 dans laquelle R¹ et R² sont -COOH, X est -CH₂-CH₂, R³ représente H, R⁴ et R⁵ comprennent chacun un groupement méthoxyphénanthrolinyle portant un groupe A⁴ carboxyle, les substituants R⁴ et R⁵ correspondant à la formule (VI) ci-après :

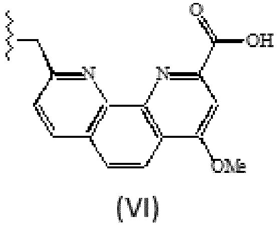

3. Composé selon la revendication 1 ou 2, **caractérisé en ce qu'**il est sous forme cationique, l'azote porteur des substituants R⁴ et R⁵, ainsi qu'éventuellement les hétéroatomes de ces substituants, se trouvant sous forme protonée.

4. Composé selon la revendication 1 ou 2, **caractérisé en ce qu'**il est sous forme anionique, les différents groupes Aⁱ se présentant sous forme de sels.

5. Composé selon la revendication 1 ou 2, **caractérisé en ce qu'**il est sous forme zwitterionique, l'azote porteur des substituants R⁴ et R⁵, ainsi qu'éventuellement les hétéroatomes de ces substituants, se trouvant sous forme protonée, et les différents groupes Aⁱ se présentant sous forme de sels.

6. Procédé de préparation d'un complexe de lanthanide, **caractérisé en ce qu'**il consiste à faire réagir un composé (I) selon l'une quelconque des revendications précédentes avec un composé donneur de cation lanthanide.

7. Procédé selon la revendication 6, **caractérisé en ce que** le composé donneur de cation lanthanide est choisi parmi les halogénures de lanthanide hydratés, les nitrates de lanthanide hydratés, les carbonates de lanthanide et les triflates de lanthanide.

8. Procédé selon la revendication 6, **caractérisé en ce que** la réaction est effectuée en solution dans un solvant choisi parmi l'eau, le méthanol, l'éthanol ou l'acétonitrile.

9. Procédé selon la revendication 6, **caractérisé en ce que** l'on fait réagir le composé (I) avec le précurseur de l'ion lanthanide dans un mélange de méthanol et d'eau à un pH allant de 3 à 5, pendant une durée comprise entre 10 minutes et 24 heures, à une température comprise entre 25°C à 80°C, puis l'on ajuste le pH de la solution à 7,0 et on évapore le méthanol.

10. Complexe obtenu par un procédé selon la revendication 6, constitué par un ion lanthanide Ln complexé par un ligand qui répond à la formule (I) telle que définie à l'une des revendications 1 ou 2.

11. Complexe selon la revendication 10, **caractérisé en ce que** l'ion lanthanide est choisi parmi les ions europium, terbium, samarium, dysprosium, erbium, ytterbium, néodyme et gadolinium.

12. Complexe selon la revendication 10, **caractérisé en ce que** le substituant R⁴ du composé (I) forme 3 cycles chélates avec le cation lanthanide.

13. Complexe selon la revendication 10, **caractérisé en ce que** le substituant R⁵ forme 3 cycles chélates à 5 membres avec le cation lanthanide.

14. Procédé pour l'analyse quantitative ou qualitative d'un composé, **caractérisé en ce qu'**il consiste à lier de manière covalente audit composé, un marqueur constitué par un complexe selon l'une des revendications 10 à 13, et à détecter ou quantifier la présence du composé marqué grâce aux propriétés de luminescence du marqueur.

15. Procédé selon la revendication 14, **caractérisé en ce que** le complexe est un complexe d'europium, de terbium, de samarium ou de dysprosium.

16. Procédé selon la revendication 14, **caractérisé en ce que** le substituant R¹ du complexe est choisi parmi les groupes carboxyle ou ester N-succinimidique.

17. Agent de relaxation pour la résonnance magnétique nucléaire, constitué par un complexe selon l'une des revendications 10 à 13.

18. Agent de relaxation selon la revendication 17, **caractérisé en ce qu'**il est constitué par un complexe de gadolinium, d'europium ou de dysprosium.

## Patentansprüche

1. Verbindung der Formel (I): 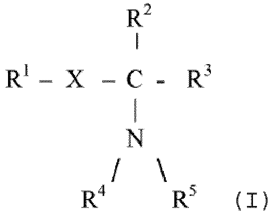 in kationischer, zwitterionischer oder anionischer Form, wobei
- R¹ eine Gruppe ist, ausgewählt aus den Gruppen Carboxyl, N-Succimidester und Sulfosuccimidester;
- X eine einfache Bindung oder eine Alkylengruppierung mit 2 Kohlenstoffatomen darstellt,
- R² eine anionische Gruppierung mit neutralem pH-Wert A² ist, ausgewählt aus den Gruppen CO₂H, SO₃H, P(O)(OR)OH, P(O)R(OH) und P(O)(OH)₂, wobei R eine Alkylgruppe oder eine Arylgruppe ist;
- R³ H darstellt:
- der Substituent R⁴ eine 2,2'-Bipyridylgruppierung umfasst, die eine Gruppe A⁴ trägt, ausgewählt aus einem Carboxyl, einem Monoalkylphosphonat, einem Monoarylphosphonat und einem Phosponyl, oder eine Phenanthrolinylgruppierung, die eine Carboxylgruppe A⁴ trägt, wobei der Substituent R⁴ eine der folgenden Formeln (II), (III), (IV) und (V) ist: 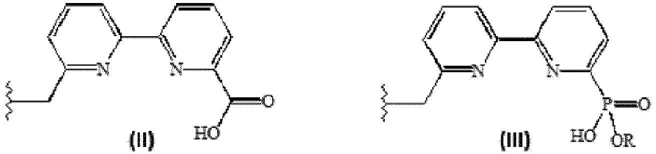 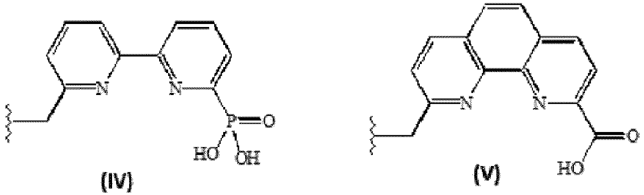
wobei R Aryl oder Alkyl ist;
- der Substituent R⁵ identisch mit R⁴ ist, wobei jeder der Substituenten R⁴ und R⁵ ein monovalenter Substituent ist, wobei die Substituenten R⁴ und R⁵ zusammen keine divalente Gruppe bilden, und
- in den Substituenten R², R⁴ und R⁵, R eine Alkylgruppe mit C₁ bis C₃ oder eine Arylgruppe mit C₅ bis C₉ ist.

2. Verbindung der folgenden Formel (I): 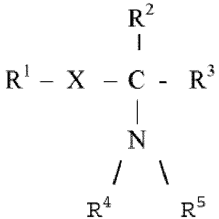 wobei R¹ und R² -COOH sind, X CH₂-CH₂ ist, R³ H darstellt, R⁴ und R⁵ jeweils eine Methoxyphenanthrolinylgruppierung umfassen, die eine Carboxylgruppe A⁴ trägt, wobei die Substituenten R⁴ und R⁵ der folgenden Formel (VI) entsprechen:

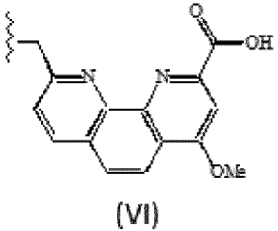

3. Verbindung nach Anspruch 1 oder 2, **dadurch gekennzeichnet, dass** sie eine kationische Form hat, wobei der tragende Stickstoff der Substituenten R⁴ und R⁵ sowie eventuell die Heteroatome dieser Substituenten protonierte Form aufweisen.

4. Verbindung nach Anspruch 1 oder 2, **dadurch gekennzeichnet, dass** sie eine anionische Form hat, wobei die verschiedenen Gruppen Aⁱ die Form von Salzen darstellen.

5. Verbindung nach Anspruch 1 oder 2, **dadurch gekennzeichnet, dass** sie eine zwitteronische Form hat, wobei der tragende Stickstoff der Substituenten R⁴ und R⁵ sowie eventuell die Heteroatome dieser Substituenten eine protonierte Form aufweisen und wobei die verschiedenen Gruppen Aⁱ die Form von Salzen darstellen.

6. Verfahren zur Herstellung eines Lanthanidkomplexes, **dadurch gekennzeichnet, dass** es darin besteht, eine Verbindung (I) nach einem beliebigen der vorhergehenden Ansprüche mit einer Donorverbindung von Lanthanidkation zu reagieren.

7. Verfahren nach Anspruch 6, **dadurch gekennzeichnet, dass** die Donorverbindung von Lanthanidkation ausgewählt ist aus den hydratisierten Lanthanidhalogeniden, den hydratisierten Lanthanidnitraten, den Lanthanidcarbonaten und den Lanthanidtriflaten.

8. Verfahren nach Anspruch 6, **dadurch gekennzeichnet, dass** die Reaktion in Lösung in einem Lösemittel durchgeführt wird, ausgewählt aus Wasser, Methanol, Ethanol oder Acetonitril.

9. Verfahren nach Anspruch 6, **dadurch gekennzeichnet, dass** die Verbindung (I) mit dem Vorläufer des Lanthanidions in einer Mischung aus Methanol und Wasser mit einem pH-Wert, der von 3 bis 5 reicht, während einer Dauer im Bereich zwischen 10 Minuten und 24 Stunden bei einer Temperatur im Bereich zwischen 25 °C und 80 °C reagiert wird, dann der pH-Wert der Lösung auf 7,0 eingestellt und das Methanol verdampft wird.

10. Kompex, erhalten durch ein Verfahren nach Anspruch 6, bestehend aus einem Lanthanidion-Ln-Komplex durch einen Liganden der Formel (I), wie in einem der Ansprüche 1 oder 2 definiert.

11. Komplex nach Anspruch 10, **dadurch gekennzeichnet, dass** das Lanthanidion ausgewählt ist aus den Ionen Europium, Terbium, Samarium, Dysprosium, Erbium, Ytterbium, Neodym und Gadolinium.

12. Komplex nach Anspruch 10, **dadurch gekennzeichnet, dass** der Substituent R⁴ der Verbindung (I) 3 Chelatzyklen mit dem Lanthanidkation bildet.

13. Komplex nach Anspruch 10, **dadurch gekennzeichnet, dass** der Substituent R⁵ 3 Chalatzyklen mit 5 Gliedern mit dem Lanthanidkation bildet.

14. Verfahren zur quantitativen oder qualitativen Analyse einer Verbindung, **dadurch gekennzeichnet, dass** es darin besteht, auf kovalente Weise an die Verbindung einen Marker zu binden, der aus einem Komplex nach einem der Ansprüche 10 bis 13 besteht, und die Anwesenheit der markierten Verbindung dank den lumineszenten Eigenschaften des Markers nachzuweisen oder zu quantifizieren.

15. Verfahren nach Anspruch 14, **dadurch gekennzeichnet, dass** der Komplex ein Komplex aus Europium, Terbium, Samarium oder Dysprosium ist.

16. Verfahren nach Anspruch 14, **dadurch gekennzeichnet, dass** der Substituent R¹ des Komplexes ausgewählt ist aus den Carboxyl- oder N-Succimidgruppen.

17. Mittel zur Entspannung für die Nuklearmagnetresonanz, das aus einem Komplex nach einem der Ansprüche 10 bis 13 besteht.

18. Mittel zur Entspannung nach Anspruch 17, **dadurch gekennzeichnet, dass** es aus einem Komplex aus Gadolinium, Europium oder Dysprosium besteht.

## Claims

1. A compound fitting formula (I): 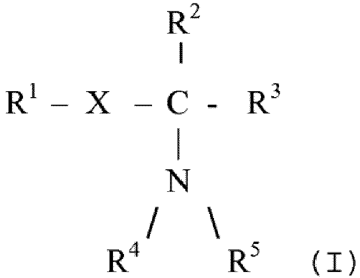 in a cationic, zwitterionic or anionic form,
wherein:
- R¹ is a group selected from among carboxyl, N-succinimidic ester and sulfosuccinidic groups;
- X represents a simple bond or an alkylene group having 2 carbon atoms;
- R² is an anionic neutral pH group A² selected from among the CO₂H, SO₃H, P(O)(OR)OH, P(O)R(OH) and P(O)(OH)₂ groups wherein R is an alkyl group or an aryl group;
- R³ represents H;
- the substituent R⁴ comprises a 2,2'-bipyridyl group bearing a group A⁴ selected from among a carboxyl, a monoalkylphosphonate, a monoarylphosphonate and a phosphonyl, or a phenanthrolinyl group bearing a carboxyl A⁴ group, the substituent R4 corresponding to one of the following formulae (II), (III), (IV) and (V): 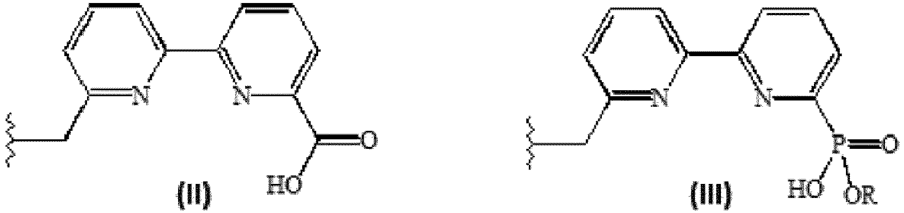 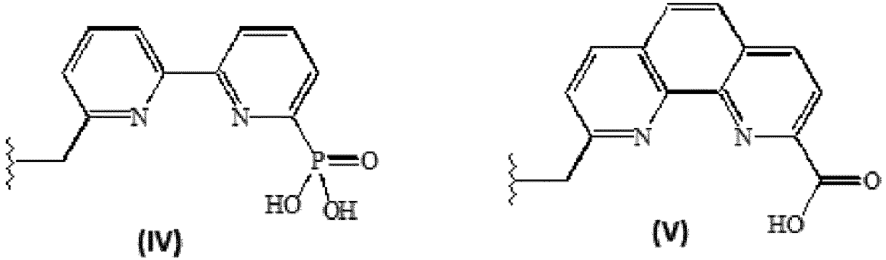
R being an aryl or an aryl;
- the substituent R⁵ is identical with R⁴, each of the substituents R⁴ and R⁵ being a monovalent substituent,the substituents R⁴ and R⁵ not forming together a divalent group, and
- in the substituents R², R⁴ and R⁵, R is a C₁-C₃ alkyl group or an C₅-C₉ aryl group.

2. A compound fitting formula (I): 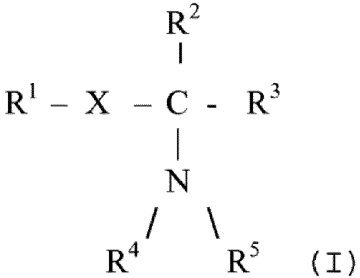 wherein R¹ and R² are -COOH, X is -CH₂-CH₂, R³ represents H, R⁴ and R⁵ each comprise a methoxyphenanthrolinyl bearing a carboxyl A⁴ group, the substituents R⁴ and R⁵ corresponding to the formula (VI) hereafter:

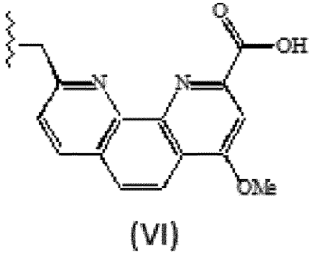

3. The compound according to claim 1 or 2, **characterized in that** it is in a cationic form, the nitrogen bearing the substituents R⁴ and R⁵, as well as optionally the heteroatoms of these substituents, found in a protonated form.

4. The compound according to claim 1 or 2, **characterized in that** it is in an anionic form, the different groups Aⁱ appearing as salts.

5. The compound according to claim 1 or 2, **characterized in that** it is in a zwitterionic form, the nitrogen bearing the substituents R⁴ and R⁵, as well as optionally the heteroatoms of these substituents, found in a protonated form, and the different groups Aⁱ appearing as salts.

6. A method for preparing a lanthanide complex, **characterized in that** it consists of reacting a compound (I) according to any of the preceding claims, with a lanthanide cation donor compound.

7. The method according to claim 6, **characterized in that** the lanthanide cation donor compound is selected from among lanthanide halide hydrates, lanthanide nitrate hydrates, lanthanide carbonates and lanthanide triflates.

8. The method according to claim 6, **characterized in that** the reaction is carried out in solution in a solvent selected from among water, methanol, ethanol or acetonitrile.

9. The method according to claim 6, **characterized in that** the compound (I) is reacted with the precursor of the lanthanide ion in a mixture of methanol and water at a pH ranging from 3 to 5, for a period comprised between 10 minutes and 24 hours, at a temperature comprised between 25°C and 80°C, and then the pH of the solution is adjusted to 7.0 and then the methanol is evaporated.

10. The compound obtained by the method according to claim 6, formed with a lanthanide ion Ln complexed with a ligand which fits formula (I) as defined in one of claims 1 or 2.

11. The complex according to claim 10, **characterized in that** the lanthanide ion is selected from among the europium, terbium, samarium, dysprosium, erbium, ytterbium, neodymium and gadolinium ions.

12. The complex according to claim 10, **characterized in that** the substituent R⁴ of the compound (I) forms 3 chelate rings with the lanthanide cation.

13. The complex according to claim 10, **characterized in that** the substituent R⁵ forms 3 chelate rings with 5 members with the lanthanide cation.

14. A method for quantitative or qualitative analysis of a compound, **characterized in that** it consists of covalently binding to said compound, a marker made up by a complex according to one of claims 10 to 13, and of detecting or quantifying the presence of the marked compound by the luminescence properties of the marker.

15. The method according to claim 14, **characterized in that** the complex is an europium, terbium, samarium or dysprosium complex.

16. The method according to claim 14, **characterized in that** the substituent R¹ of the complex is selected from among the carboxyl and N-succinimide ester groups.

17. A relaxation agent for nuclear magnetic resonance, formed by a complex according to one of claims 10 to 13.

18. The relaxation agent according to claim 17, **characterized in that** it is formed by a gadolinium, europium or dysprosium complex.
